# EUROPEAN PATENT APPLICATION

(11) **EP 3 916 098 A1**
(43) Date of publication of application: **01.12.2021**
(21) Application number: 20176378.6
(22) Date of filing: 25.05.2020
(51) Int. Cl.: C12N 15/861, A61K 35/761

(54) **ADENOVIRUS FOR ANTI-TUMOUR THERAPY**

(71) Applicant: Medizinische Hochschule Hannover, 30625 Hannover (DE)
(72) Inventor: Kühnel, Florian, 30163 Hannover (DE); Woller, Norman, 30629 Hannover (DE); Kubicka, Stefan, 72074 Tübingen (DE)
(74) Representative: Taruttis, Stefan Georg

(57) **Abstract**

Adenovirus for use in anti-tumour therapy which has specificity for tumour cells, especially for tumour cells that have reduced activity levels of the tumour suppressor p53. It is known that the majority of tumours characteristically exhibit reduced activity of the tumour repressor p53. In tumour-bearing patients, the adenovirus provides for an effective cross-presentation of tumour antigens, which can be neoantigens, e.g. having low immunogenicity, and hence supports the induction of tumour-specific immune cells, especially of tumour-specific CD8 T-cells. Further, the adenovirus in tumour patients stimulates the non-specific immune response by NK-cells, preferably both locally and systemically, and the adenovirus improves migration of immune cells, e.g. T-cells, NK-cells, and antigen-presenting cells (APC), e.g. dendritic cells, into the tumour, and the adenovirus improves the maturation of immune cells, especially of APC, and improves the cytolysis of tumour cells by immune cells. The adenovirus has an E4 protein which contains an E4 orf4-encoded protein of one of amino acid sequences SEQ ID NO: 1, SEQ ID NO: 2, and SEQ ID NO: 3.

## Description

The present invention relates to an adenovirus for use in anti-tumour therapy. The adenovirus has specificity for tumour cells, especially for tumour cells that have reduced activity levels of the tumour suppressor p53. It is known that the majority of tumours characteristically exhibit reduced activity of the tumour repressor p53. In tumour-bearing patients, the adenovirus provides for an effective cross-presentation of tumour antigens, which can be neoantigens, e.g. having low immunogenicity, and hence supports the induction of tumour-specific immune cells, especially of tumour-specific CD8 T-cells. Further, the adenovirus in tumour patients stimulates the non-specific immune response by NK-cells, preferably both locally and systemically, and the adenovirus improves migration of immune cells, e.g. T-cells, NK-cells, and antigen-presenting cells (APC), e.g. dendritic cells, into the tumour, and the adenovirus improves the maturation of immune cells, especially of APC, and improves the cytolysis of tumour cells by immune cells. Further, the adenovirus in a tumour patient provides for a tumour environment which supports the immune response, e.g. by inducing expression of cytokines, e.g. IFN, in the environment of the tumour. The tumour can be a single-cell tumour or a solid tumour.

### State of the art

Kühnel et al., Cancer Gene Therapy 28-40 (2004), describe a mutated CMV-promoter (CMVgal) which in the presence of p53 is transcriptionally repressed by a synthetic fusion protein (GAL4-KRAB). The fusion protein GAL4-KRAB which represses the CMVgal-promoter is expressed under the control of a p53-dependent promoter. As a result, the CMV-promoter is active in the absence of p53, making it suitable for use in tumour-specific adenoviral gene therapy.

Gürlevik et al., Nucl Acid Res Vol. 37, No. 12 (2009), describe an adenovirus expressing interfering RNA under the control of a p53-dependent promoter (prMinRGC) to inhibit expression of essential adenoviral genes, e.g. E1A, E1B, E4, pTP, in the presence of p53, e.g. in p53-normal, non-tumour cells. The adenovirus was shown not to replicate in p53-positive cells but to replicate in p53-negative cells representing tumour cells.

Kühnel et al., Molecular Therapy 936-946 (2010), describe a chemotherapy in combination with an adenovirus that is p53-sensitive as it contains the CMVgal promoter which is repressed by a fusion protein GAL4-KRAB, which is expressed under the control of a p53-denpendent promoter (prMinRGC).

### Object of the invention

It is an object of the invention to provide an adenovirus that is suitable for use in tumour treatment and which is specific for tumour tissue and induces an effective CD8 T-cell immune response, and which adenovirus preferably also effectively induces an immune response directed against the tumour rather than an immune response directed against the adenovirus. More preferably, the adenovirus is intended to induce an effective immune response against a tumour bearing a tumour antigen that inherently is not highly immunogenic, e.g. a neo-antigen.

### Description of the invention

The invention achieves the object by the features of the claims and especially provides adenovirus, especially for use in the treatment of a tumour, the adenovirus having an E4 protein which contains an E4 orf4-encoded protein of one of amino acid sequences 
MVLPSLPSPLLLETQSSCIAWLGFAYATVDDFLRTIRCDGVLITTEASILLTNLRVWLY
LNYHTEHAKRQDRRRRSVCHARTWFCFRKYDYVRRSIWHDTTTNTISVVSAHSVQ
(SEQ ID NO: 1),
 
MILPSLPSPLLLETQSSCIAWLGFAYATVDDFLRTIKYDEVLITTEASILLTNLRVWLY
LNYQTEHTKRQDRRRRSVCHARTWFCFRKYDYVRRSIWHDTTTNTISVVSAHSVQ
(SEQ ID NO: 2), and
 MILPSLPSPLLLETQSSCIAWLGFAYATVDDFLRTIKYDGVLITTEASILLTNLRVWLY LNYQTEHTKRQDRRRRSVCHARTWFCFRKYDYVRRSIWHDTTTNTISVVSAHSVQ (SEQ ID NO: 3), including an amino acid sequence having a sequence identity or homology of at least 80%, of at least 85%, of at least 90%, of at least 95% to one of, to two of, or to the three of the sequences SEQ ID NO: 1, SEQ ID NO: 2 and SEQ ID NO: 3,
and a DNA encoding an adenovirus containing the coding sequence for an E4 protein containing the E4 orf4 encoded protein.

For the adenovirus for use in the treatment of a tumour, the tumour can express a neo-antigen, which can e.g. have a low antigenicity, because the adenovirus provides for an effective cross-presentation of tumoural antigens to immune cells.

The E4 protein according to the invention comprises or consists of the protein sections encoded by E4 orf1, E4 orf2, E4 orf3, E4 orf4 having one of SEQ ID NO: 1, SEQ ID NO: 2, and SEQ ID NO: 3, E4 orf6 and E4 orf7, wherein E4 orf3 preferably is inactivated, and wherein preferably E4 orf1, E4 orf2, E4 orf3, E4 orf6 and E4 orf7 have the amino acid sequence of the respective wild-type Ad5. A protein section encoded by an inactivated E4 orf3 may be an absent E4 orf3 protein section or a truncated E4 orf3 protein section, e.g. consisting of at maximum N-terminal 10 amino acids, preferably of at maximum N-terminal 7 amino acids or less of the wild-type E4 orf3 encoded protein section.

Further, the invention provides a process for producing the adenovirus of the invention, which process has the advantage of producing the adenovirus at a high titer, because the E4 protein containing one of the E4 orf4 sequences of SEQ ID NO: 1 to NO: 3 does not result in a lower titer, e.g. compared to adenovirus containing the wild-type E4 or compared to wild-type Ad5. Further, it has been found that the adenovirus according to the invention has a high stability e.g. for biotechnological production of the virus.

The E4 encoding sequence preferably contains or consists of the wild-type sequences of Ad5 for orf1, orf2, orf3 or inactivated orf3, an E4 protein containing the orf4 of one of SEQ ID NO: 1, NO: 2 or NO: 3, and wild-type sequences of Ad5 for orf6 and orf7.

It was found that an adenovirus, which preferably is adenovirus serotype 5 (Ad5), comprising in its E4 protein an E4-orf4 encoded protein section of one of SEQ ID NO: 1, SEQ ID NO: 2, and SEQ ID NO: 3, and preferably an inactivated E4 orf3 protein section, has high specificity for cells with reduced activity levels of p53, which is e.g. a reduced or absent expression of p53, e.g. for p53-negative tumour cells, induces an effective immune response, e.g. an effective CD8 T-cell response, which is directed against tumour antigen, and which immune response is less anti-viral e.g. compared to wild-type Ad5, wherein the anti-tumour immune response can also be directed against a tumour neo-antigen that has a low immunogenicity.

In addition, the adenovirus of the invention has been found to be less hepatotoxic, e.g. compared to wild-type Ad5.

Preferably, the virus of the invention in its nucleic acid sequence contains an expression cassette encoding an effector molecule for expression in tumour cells. The expression cassette encoding an effector molecule can e.g. be under the control of an IRES (internal ribosomal entry site) or under the control of a separate promoter, e.g. a promoter that is inactivated by the presence of p53. The effector molecule can comprise or consist of at least one effector molecule, e.g. a fusion protein of two or three effector molecules, which preferably are connected by an intermediate 2A element. Exemplary effector molecules are cytokines, e.g. FLT3L (FMS-like tyrosine kinase 3 ligand), MIP1a (macrophage inflammatory protein 1a), XCL1 (chemokine (C Motif) ligand).

Generally preferred, the adenovirus of the invention contains a double-stranded nucleic acid molecule in which the arrangement of the genetic elements corresponds to their arrangement in the wild-type Ad5, more preferably with an expression cassette for an effector molecule arranged directly adjacent to the 3' terminus of coding region for E1B, and directly adjacent to the 3' terminus of the expression cassette for an effector molecule an expression cassette for inhibitory RNA directed against the E1A encoding DNA sequence, against the E1B encoding DNA sequence, against the E1B promoter, against the TP encoding DNA sequence, against the Pol encoding sequence, and/or against the E4 encoding DNA sequence.

Preferably, the adenovirus encoding DNA from 5' to 3' comprises or consists of a left-end inverted terminal repeat (L-ITR), a first promoter, a coding sequence for E1A, an E1B promoter controlling a coding sequence for E1B, an expression cassette for at least one effector molecule, preferably an expression cassette encoding inhibitory RNA (RNAi) that are specific for RNAi target sites within the E1A encoding sequence and/or within the E1B promoter and/or within the E1B encoding sequence, vector backbone sequence, and on the opposite DNA strand an expression cassette for E4 comprising the orf4 of one of SEQ ID NO: 1, NO: 2 and NO: 3, the expression cassette for E4 containing in functional relationship a promoter driving expression of E4, and a right-end inverted terminal repeat (R-ITR). The arrangement of the L-ITR, E1A, a promoter controlling expression of E1B, preferably of an expression cassette for at least one effector molecule on one DNA strand, and preferably of coding sequences for inhibitory RNA under the control of a promoter which is activated by the presence of p53, e.g. the prMinRGC promoter, on one DNA strand, and arrangement of the expression cassette for E4 on the opposite DNA strand corresponds to the arrangement of the R-ITR, E4-promoter, and of the orfs constituting the coding sequence for E4 from 5' to 3' on the opposite strand. The expression cassette for an E4-orf4 encoded protein is arranged on the DNA strand opposite to the strand encoding the L-ITR, the first promoter, the E1A encoding sequence and the E1B promoter and the E1B encoding sequence. The L-ITR is arranged at one terminus of the DNA and the R-ITR is arranged at the terminus of the DNA opposite of the L-ITR.

The first promoter preferably is CMVs, which has the advantage of becoming activated following infection of a cell with a time delay that allows for the effective inhibition of the E1A encoding sequence, the E1B promoter and of the E1B encoding sequence by inhibitory RNA molecules that are expressed under the control of prMinRGC.

Preferably, the coding sequence for E1A, the E1B promoter, and the coding sequence for E1B contain RNAi target sites for binding inhibitory RNA, e.g. shRNA, and the adenovirus encoding DNA contains an expression cassette for inhibitory RNA, e.g. for shRNA, which bind to the RNAi target sites, which expression cassette preferably is under the control of a promoter which is activated by presence of p53, e.g. the promoter prMinRGC.

The coding sequences for inhibitory RNA, which inhibit expression of the adenoviral genes, e.g. of E1A, E1B, E4, pTP, Pol, are preferably expressed under the control of the prMinRGC promoter. The prMinRGC promoter is preferred as the promoter which is activated by the presence of p53. The inhibitory RNA and their target sequences preferably are those described by Gürlevik et al. (2009), quoted above.

In the alternative or in addition to the combination of RNAi arranged under the control of the prMinRGC promoter and CMVs as the first promoter, the first promoter can be a CMV promoter (CMVgal) which in the presence of p53 is transcriptionally repressed by a synthetic fusion protein (GAL4-KRAB), and the DNA encoding the adenovirus comprising an expression cassette encoding the fusion protein GAL4-KRAB under the control of a promoter that is activated by presence of p53. This embodiment also provides for a tight control of expression of E1A and E1B such that these proteins are effectively repressed in the presence of p53 in an infected cell, but that these proteins are effectively expressed in a target cell having reduced activity levels of p53. The CMVgal promoter and the GAL4-KRAB preferably are those described by Kühnel et al., Cancer Gene Therapy 28-40 (2004), quoted above.

The coding sequence for E1A preferably encodes E1A of Ad5, in which amino acids No. 4 to No. 25 of the wild-type sequence are deleted, e.g. amino acids No. 727 - 1646 of SEQ ID NO: 4. In the viral DNA of SEQ ID NO: 4, the E4 orf4 encoded protein of the invention is contained in reverse complementary orientation, as it is usual for the Ad5. The E4 region is read from the promoter adjacent the left ITR, i.e. from right to left, also reversing the way of numbering E4 genes. The E4 orf4 is encoding DNA section contains splice sites, and therefore the reverse complementary sequence does not directly present codons.

The vector backbone sequence comprises or consists of the following elements, e.g. from 5' to 3' DNA-binding protein (dbp), terminal protein (TP), polymerase (Pol), Penton (Pent), Hexon (Hex), and Fiber (Fiber).

Due to the specificity of the adenovirus for tumour cells, which are preferably characterized by lower content of p53 than average normal cells, e.g. non-tumour cells, or absence of p53, or presence of dysfunctional p53, the adenovirus is suitable for use in the treatment of a tumour, preferably of a tumour that has a lower presence or lower expression of p53 or an absent presence or absent expression of p53, or presence of dysfunctional p53 e.g. in comparison to non-tumour cells. The specificity of the adenovirus for tumour cells, which especially are p53-deficient cells, in combination with the inhibition of the expression of E1A, E1B in the presence of p53, and the adenovirus preferably containing an expression cassette for at least one effector molecule in cells that express p53 at normal levels makes the adenovirus of the invention suitable for use in the treatment of a tumour.

The adenovirus of the invention is also for use in the treatment of a tumour in combination with anti-PD1/PD-L1 immune therapy. Anti-PD1/PD-L1 immune therapy can be by e.g. Nivolumab, Pembrolizumab, Atezolizumab or a combination of at least two of these.

In the figures
- Fig. 1 schematically shows the elements of a preferred embodiment of the adenovirus of the invention,
- Fig. 2 schematically shows the elements of a further embodiment of the adenovirus of the invention,
- Fig. 3 a) to h) shows light-microscopic pictures of cultivated cells after infection with adenovirus,
- Fig. 4 shows the replication kinetics of an adenovirus of the invention and of a comparative adenovirus,
- Fig. 5 shows ELISA results for expression of an effector molecule from different cells infected with an embodiment of the adenovirus of the invention,
- Fig. 6 shows the results of an ELISPOT assay after tumour treatment using an adenovirus of the invention,
- Fig. 7 a) to e) shows results of an analysis of the immune response against tumour bearing neo-antigens after use of an adenovirus of the invention, and
- Fig. 8 a) to e) show the results of CsCl gradient separation of defect and intact adenovirus.

### Example: Generating an adenovirus having higher specificity for p53-deficient cells

As an example of tumour cells, the p53-deficient H1299 lung carcinoma cells were used as host cells, which were co-infected with adenovirus Ad5, adenovirus Ad4, and co-infected with adenovirus Ad11, or other alternative serotypes of adenovirus, in the presence of the mutagen ethylmethyl sulfonate. As an alternative, the p53-deficient H1299 lung carcinoma cells were co-infected with adenovirus Ad3, adenovirus Ad5, and optionally additionally co-infected with Ad11 and Ad 21, in the presence of the mutagen ethylmethyl sulfonate. The adenovirus Ad5 contained the expression cassette for inhibitory RNA as described by Gürlevik et al. (2009) under the control of the p53-dependent promoter prMinRGC, rendering the expression of E1A and E1B dependent on the presence of p53, and hence rendering the Ad5 dependent on p53, respectively dependent on healthy p53 cellular levels. Following complete lysis of the host cells by viruses, the viruses were passaged to newly seeded host cells. This was repeated for 12 cycles of passaging after complete lysis. The resulting virus samples were diluted to limitation for isolating single clones, which were purified. For identification of possible genetic alterations of the viral clones, two viral DNA sections were amplified by PCR and the amplificates were tested by digestion with restriction enzymes cutting in wild-type Ad4 and in wild-type Ad11, but not in the Ad5. This restriction fragment analysis identified virus clones that were subsequently sequenced and showed a coding sequence for an E4 orf4 of SEQ ID NO: 1, of SEQ ID NO: 2, and of SEQ ID NO: 3, respectively. In addition, it was found in some clones that the E4 orf3 was inactivated by an insertion mutation generating a stop codon followed by a frameshift, truncating the transcript of E4 orf3 to 7 amino acids.

Further analysis revealed that these E4 proteins containing an E4 orf4 of SEQ ID NO: 1, of SEQ ID NO: 2, or of SEQ ID NO: 3 are hybrid E4 orf4 protein sections of an N-terminal portion from the E4 orf4 of Ad11 with a C-terminal portion from the E4 orf4 from Ad5 (SEQ ID NO: 1), or of an N-terminal portion from the E4 orf4 of Ad3 with a C-terminal portion from the E4 orf4 from Ad5 (SEQ ID NO: 2), or of an N-terminal portion from the E4 orf4 of Ad21 with a C-terminal portion from the E4 orf4 from Ad5 (SEQ ID NO: 3).

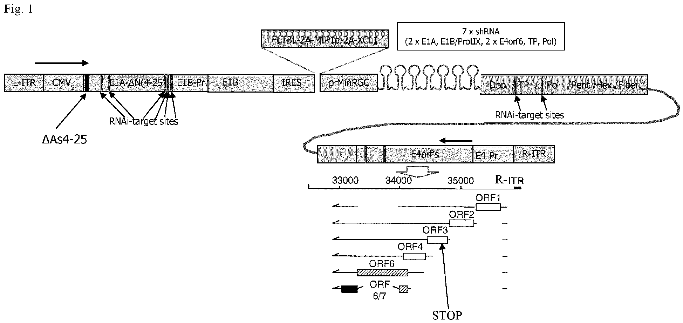

In order to generate a preferable adenovirus suitable for the treatment of a tumour, a schematic of which is shown in Fig. 1, the Ad5 containing one of the E4 orf4 in its E4 encoding gene was additionally provided with an expression cassette encoding at least one effector molecule, exemplified in Fig. 1 by a coding sequence for a fusion protein FLT3LK-2A-MIP1a-2A-XCL1 in which the single effector molecules are separable by the intermediate 2A element, or exemplified by the coding sequence for human FLT3L (hFLT3L) under the control of an IRES sequence, each optionally followed by a poly adenylation signal (pA). The expression cassette for the at least one effector molecule was arranged directly adjacent to the 3'-terminus of the E1B encoding sequence. The expression cassette for inhibitory RNA (shRNA) under the control of the p53-dependent promoter prMinRGC (minRGC) was arranged in 3' directly adjacent the expression cassette encoding at least one effector molecule.

Further, in the Ad5, the wild-type promoter of the E1A was replaced by a shortened CMV promoter (CMVs having 245 bp). The CMVs promoter starts transcription of the viral elements that are arranged under its control, which is seen as an advantage especially in combination with the expression cassette for inhibitory RNA directed against the viral elements which are arranged under the control of the CMVs promoter. The viral elements arranged under the control of the CMVs promoter from 5' to 3' are the coding sequence for E1A, the E1B promoter and the coding sequence for E1B (E1A-ΔN(4-25)-E1B-Pr.-E1B), and preferably the expression cassette for the at least one effector molecule. Therein, the E1A encoding sequence as preferred is devoid of the N-terminal amino acids No. 4 to No. 25 (ΔN(4-25)) of the wild-type E1A.

The target sequences of the inhibitory RNA molecules are E1A, E1B, dbp, TP and Pol, the target sites (RNAi-target sites) are indicated.

The viral elements which in Fig. 1 are designated as Backbone preferably have their wild-type arrangement and their wild-type sequence of Ad5 and are arranged between the expression cassette encoding the inhibitory RNA molecules and the E4 encoding sequence.

These elements are arranged between a left-end inverted terminal repeat (L-ITR) and a righthand inverted terminal repeat (R-ITR).

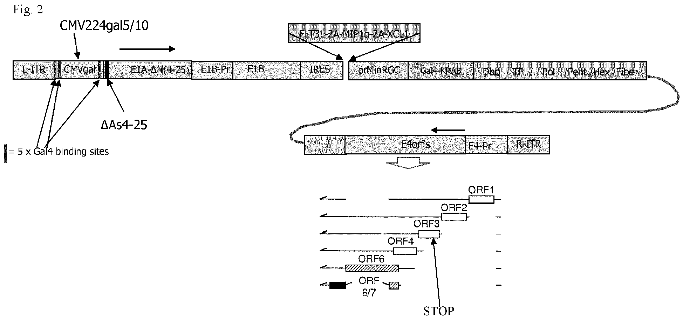

In Fig. 1 and Fig. 2, the orfs of E4 are schematically depicted in one DNA molecule comprising the adenovirus, and, in accordance with the arrangement on the opposite strand, the orfs of E4 are transcribed from the right to left, as indicated by the arrows.

In the coding sequence for E4, the orf4 encodes one of SEQ ID NO: 1, SEQ ID NO: 2, and SEQ ID NO: 3, and preferably, the orf3 is inactivated, e.g. by a stop codon (STOP) generated close to the N-terminus of orf3, resulting in e.g. an orf3 encoding at maximum the N-terminal 8 or less amino acids of orf3. In Fig. 1, the E4 promoter (E4-Pr.) the coding sequences for E4 are shown from left to right as they are arranged together with the R-ITR on the complementary DNA strand. The numbers indicate the length in bp.

SEQ ID NO: 4 gives the DNA sequence of this exemplary Ad5, with the nucleotide sections for:

| element | Nt sections (No ... No) |
|---|---|
| L-ITR | 1...103 |
| R-ITR | 36919...37021 |
| CMVs | 380...625 |
| E1A gene (ΔAs4-25-variant) | 727... 1646 |
| RNAi target site | 837 ... 857 |
| RNAi target site | 886 ... 909 |
| heterologous target sites for RNAi inserted into the 3'UTR of E1A, equivalent to E1A 886...909, E1A 837...857, Prot IX 7353...7374 | 1684 ... 1764 |
| Promoter for E1B | 1813 ... 1910 |
| E1B-19k | 1911...2441 |
| E1B-55k | 2216...3706 |
| IRES | 3731...4320 |
| FLT3L (cds) | 4349 ... 5053 |
| 2A | 5054 ... 5116 |
| MIP1A/CCL3 | 5117 ... 5392 |
| 2A | 5393 ... 5455 |
| XCL1 | 5456 ... 5800 |
| prMinRGC | 5872 ... 6230 |
| shRNA network (complete) | 6259 ... 7064 |
| Inhibitory shRNA for E1A (1) | 6259 ... 6321 |
| Inhibitory shRNA for E1 A (2) | 6387 ... 6444 |
| Inhibitory shRNA for Prot IX | 6511 ... 6567 |
| Inhibitory shRNA for E4orf6 (1) | 6634 ... 6690 |
| Inhibitory shRNA for E4orf6 (2) | 6756 ... 6816 |
| Inhibitory shRNA for TP | 6882 ... 6939 |
| Inhibitory shRNA for AdPol | 7006 ... 7064 |
| Protein IX, part of backbone | 7332 ...7754 |
| Polymerase; Ad-Pol, part of backbone | 8920...17842 |
| Terminal Protein; TP, part of backbone | 12306...17842 |
| Prot III: Penton, part of backbone | 17878...19593 |
| hexon, part of backbone | 22563...25421 |
| DNA-binding protein, dbd, part of backbone | 26164...27753 |
| 100k, part of backbone | 27782...30205 |
| ΔE3 (2681 bp) | at 31589 (equivalent to del. of 28137 ... 30818 in the wildtype virus) |
| fiber, part of backbone | 32108...33853 |
| E4orf6/7 | 33980...35143 |
| hybrid E4orf4, Ad5/Ad11; Seq ID NO: 1 | 35064...35408 |
| Stop codon E4orf3 | 35747...35749 |
| Inactive E4orf3 locus | 35419...35770 |
| E4orf2 | 35782...36175 |
| E4orf1 | 36207...36593 |
| r-ITR | 36919...37021 |
| Total length 37021 bp | |
| Target sequences of the shRNAs in the network | 5' to 3' : 886, 837, 7354, 34123, 34609, 13375, 10085 |

Further deviations from the sequence of Ad5, accession No. AC_000008, are indicated in the attached sequence protocol.

As an alternative to the inhibitory shRNA sections for repressing the virus in the presence of p53, i.e. in healthy cells, using a p53-dependent promoter (prMinRGC) for expressing inhibitory shRNAs against viral genes as shown in Fig. 1, a p53-dependent promoter can be used to control essential viral genes for expression only in absence of p53 in infected cells. An embodiment is shown in Fig. 2, wherein a constitutive promoter, e.g. the CMVgal promoter is arranged for expression of essential viral genes, which CMVgal promoter is repressed by a fusion protein, e.g. GAL4-KRAB, which is expressed under the control of a p53-dependent promoter, e.g. prMinRGC. In SEQ ID NO: 6, the elements of this embodiment are shown.

| element | Nt sections (No ... No) |
|---|---|
| L-ITR | 1...103 |
| CMVgal promoter (CMV224gal10/5) | 321...963 |
| E1A gene (ΔAs4-25 variant) | 1037...1956 |
| RNAi target site | (1147...1167) |
| RNAi target site | (1196...1219) |
| heterologous target sites for RNAi inserted into the 3'UTR of E1A, equivalent to E1A 1196...1219, E1A 1147...1167, Prot IX 7409...7429 | (1994...2074) |
| Promoter for E1B | 2084...2220 |
| EIB-19k | 2221...2751 |
| EIB-55k | 2526...4016 |
| IRES | 3421...4630 |
| FLT3L | 4659...5363 |
| 2A | 5364...5426 |
| MIP1A/CCL3 | 5427...5702 |
| 2A | 5703...5765 |
| XCL1 | 5766...6110 |
| prMinRGC | 6182...6538 |
| GAL4-KRAB | 6539...7144 |
| Protein IX (part of backbone) | 7388...7810 |
| Polymerase (Ad-Pol, part of backbone) | 8976...17898 |
| Terminales Protein (TP, part of backbone) | 12362...17898 |
| Prot III: Penton, part of backbone | 17935...19650 |
| Hexon, part of backbone | 22621...25479 |
| DNA-binding protein, dbd, part of backbone | 26222...27811 |
| 100k, part of backbone | 27840...30263 |
| ΔE3 (2681 bp) | at 31645 (equivalent to del. of 28137 ... 30818 in the wildtype virus) |
| Fiber, part of backbone | 32164...33909 |
| E4orf6/7 | 34036...35199 |
| hybrid E4orf4, Ad5/Ad 11; Seq ID NO: 1 | 35120...35464 |
| Stop codon E4orf3 | 35803...35805 |
| Inact. E4orf3 locus | 35475...35826 |
| E4orf2 | 35838...36231 |
| E4orf1 | 36263...36649 |
| R-ITR | 36974...37077 |
| total length 37077 bp | |

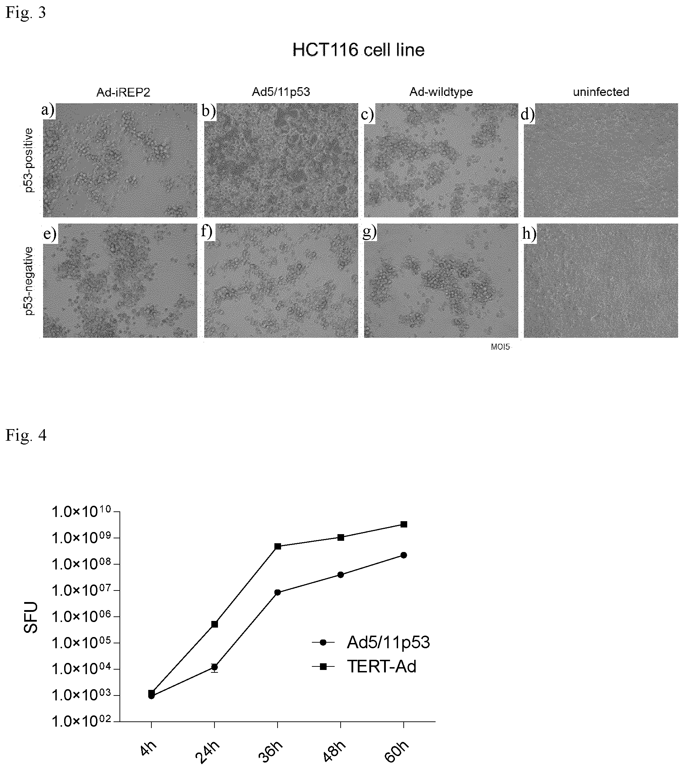

The embodiment of the viral vector of SEQ ID NO: 4 (Ad5/11p53) was used to infect the p53-positive host cell-line HCT116 and a p53-deficient but otherwise isogenic host cell-line representing a tumour cell, each with a multiplicity of infection (MOI) of 5. As a comparison, the wild-type Ad5 (Ad-wildtype) and an adenovirus having the complete wild-type E4 (Ad-iREP2) were used for infection. Fig. 3 a) to d) show microscopic pictures of the p53-positive host cell-line, and Fig. 3 e) to h) show the p53-negative host cell-line. In these pictures, non-adherent cells are cytopathic and indicate lysis. Fig. 3d) and 3 h) show the host cell-line without infection. It was found that the adenovirus of the invention in p53-negative host cells (Fig. 3 f) shows a significantly higher lysis than in p53-positive host cells (Fig 3 b), indicating its high selectivity for p53-negative cells. In comparison to comparative Ad-iREP2 and wild-type Ad5, the adenovirus of the invention showed an oncolytic effect on p53-negative cells that is comparable or higher, and which has higher specificity for p53-negative cells.

As an example of the adenovirus according to the invention, the adenovirus containing the DNA of SEQ ID NO: 4 was produced e.g. in cultivated Huh7 cells, which are p53-deficient and contain a mutation (p53del + p53Cys220), and as a comparison, adenovirus containing (hTert-Ad), and as a further comparison, wild-type Ad5. As shown in Fig. 4, the adenovirus according to the invention (Ad5/1 1p53) having SEQ ID NO: 4 in these cells had a replication kinetic that is retarded 100-fold in the first 24 h compared to hTert-Ad. The replication of the adenovirus according to the invention (Ad5/1 1p53) was retarded ca. 1000-fold in the first 24 h compared to wild-type Ad5. The retarded replication kinetics of the adenovirus according to the invention are presently considered to provide for an enhanced safety of the medical administration of the adenovirus, e.g. because the lower initial viral replication rate can result in less side-effects.

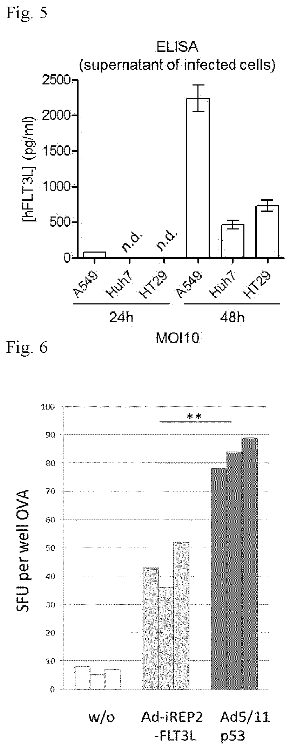

As an example of adenovirus according to the invention, an embodiment was used which corresponds to SEQ ID NO: 4 except for the effector molecule being human FLT3L (hFLT3L) only under the control of an IRES and with a poly-adenylation element at its 3'-end. As host cells, A549, Huh7 and HT29 cells, all p53-negative, were infected with a MOI of 10. The supernatant of these cell cultures was analysed by an ELISA for hFLT3L. The results, depicted in Fig. 5, show that after 48 h post infection, the effector hFLT3L is expressed from these exemplary cells.

The immunologic properties of the adenovirus of the invention was tested by infecting CMT64-Ova tumours (p53-negative) which express OVA as a model tumour antigen. The CMT64-Ova tumours were grown subcutaneously in C57BL/6 mice, and 1 x 10⁹ infectious particles of the adenovirus were injected intra-tumourally. 48 h post infection, mice were killed and splenocytes were isolated and antigen-specific (anti-OVA) T-cell responses were measured in an ELISpot assay. In the ELISpot assay, IFN-gamma antibodies were immobilised to the plate, splenocytes from C57BL/6 mice were added to the plates and stimulated with the OVA peptide SIINFEKL. After at least 24 hours of stimulation, splenocytes were removed and IFN-gamma spots were measured by HRP immunostaining.

Fig. 6 shows the results of an ELISPOT assay for 3 individual mice bearing the CMT64-Ova tumour, without infectious particles (w/o), for the comparative adenovirus having the complete wild-type E4 expressing FLT3L as the effector molecule (Ad-iREP2-FLT3L), and an embodiment of the invention (Ad5/11 p53) also expressing FLT3L and which differs from the comparative adenovirus only in the E4 orf4 encoding SEQ ID NO: 1 and E4 orf3 being inactive. The results show that the adenovirus of the invention induces a significantly higher antigen-specific CD8 T-cell response than the comparative adenovirus. This shows that the E4 orf4 of the invention effectively increases the T-cell response specifically directed against the tumour-antigen.

In a further test, a syngeneic lung tumour was used as a subcutaneous model tumour in mice. This lung tumour expresses neo-antigens, which are significantly less immunogenic than the strong immunogenic OVA antigen. These tumour-specific neoantigens were mutated forms of the genes H2-Q2, Ndufs1, Rab13, Ppat,Gsta2, Arhgef10, and Chd2 (H2-Q2-D244E, Ndufs1-V491A, Rab13-K196N, Ppat-I208M, Gsta2-Y9H, Arhgef10-M207I, and Chd2-T647A.

The analysis of the antiviral immune responses against E1A, E1B and against the adenoviral DNA-binding protein (dbp) from these mice could show that the adenovirus of the invention induced a lower immune response against viral E1B and dbp, and to a lesser extent against E1A. It was found that only the adenovirus of the invention induced significant T-cell responses directed against the tumour neo-antigens, whereas the comparative adenovirus did not induce tumour-antigen specific T-cell responses but induced stronger anti-viral immune responses.

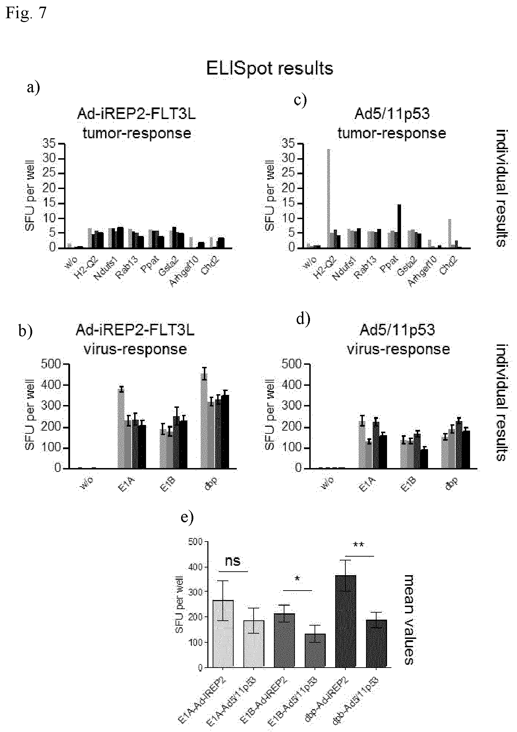

The results are shown in Fig. 7 a) and b) for the comparative adenovirus Ad-iREP2-FLT3L and in Fig. 7 c) and d) for the adenovirus according to the invention (Ad5/11p53), and Fig. 7 e) shows mean values.

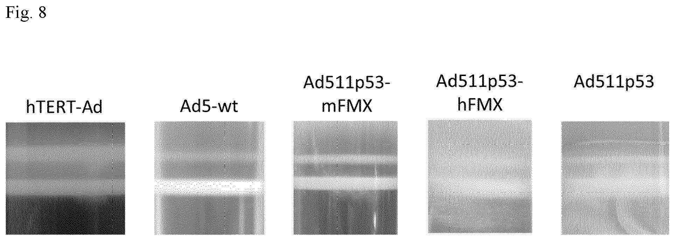

The adenovirus of the invention also has increased stability during replication, which contributes to induction of a lower anti-viral immune response and of a higher anti-tumoral immune response. The stability of the adenovirus of the invention was tested by determining the proportion of intact viral particles to defect viral particles. The expression cassette encoding the effector molecule was provided with the coding sequence for hFLT3L only, for the combination of hFLT3L-2A- CCL3-2A-XCL1, wherein also XCL1 and CCL3 had the human sequence, and for the combination of mFLT3L-2A- CCL3-2A-XCL1, wherein also XCL1 and CCL3 had the murine sequence. The stability was determined by isolating the virus from cell culture producing the adenovirus, by freeze-thaw cycles followed by CsCl ultra-centrifugation and determining the thickness of the band of intact virus (lower light band) in relation to the thickness of the band of defect virus (upper light band) as shown in Fig. 8 a) for the comparative adenovirus hTERT, b) for wild-type Ad5 (Ad5-wt), c) for an adenovirus of the invention having SEQ ID NO: 4 and encoding murine FMX (mFMX) as the effector molecule, d) for an adenovirus of the invention having SEQ ID NO: 4 or SEQ ID NO: 6 and encoding human FMX (hFMX), and in e) for an adenovirus of the invention having SEQ ID NO: 4 or SEQ ID NO: 6 and encoding only hFLT3L (Ad5/11p53) as the effector molecule. These embodiments, comprising a coding sequence for one effector molecule or a coding sequence for three effector molecules, showed a stability similar to or higher than the stability of wild-type Ad5.

## Claims

1. Adenovirus comprising an E4 protein comprising an E4 orf4 having an amino acid sequence of at least 80% homology to SEQ ID NO: 1, to SEQ ID NO: 2, and/or to SEQ ID NO: 3.

2. Adenovirus according to claim 1, **characterized in that** the E4 protein consists of E4 orf1, E4 orf2, inactivated E4 orf3, E4 orf4 having one of SEQ ID NO: 1, SEQ ID NO: 2, and SEQ ID NO: 3, E4 orf6 and E4 orf7.

3. Adenovirus according to one of the preceding claims, comprising a nucleic acid coding sequence comprising a terminally arranged left-end ITR (L-ITR), a first promoter functionally arranged at a nucleic acid encoding E1A, an expression cassette of an E1B promoter functionally arranged at the E1B encoding sequence, an expression cassette encoding an effector molecule, an E4 promoter functionally arranged at the nucleic acid sequence encoding the E4, an expression cassette encoding the E4 protein under the control of an E4 promoter, and a terminally arranged right-end ITR (L-ITR) opposite the left-end ITR (L-ITR).

4. Adenovirus according to one of the preceding claims, **characterized by** comprising at least one expression cassette for inhibitory RNA encoding inhibitory RNA directed against the E1A encoding DNA sequence, against the E1B encoding DNA sequence, against the E1B promoter, against the TP encoding DNA sequence, against the Pol encoding sequence, and/or against the E4 encoding DNA sequence, which inhibitory RNA are arranged under the control of a promoter that is activated by the presence of p53.

5. Adenovirus according to one of the preceding claims, **characterized by** comprising at least one expression cassette for viral genes comprising DNA-binding protein, terminal protein, polymerase, Penton, Hexon and Fiber, under the control of a constitutive promoter, which is repressable by a fusion protein which is expressed under the control of a p53-dependent promoter.

6. Adenovirus according to one of the preceding claims, **characterized in that** the first promoter is a CMVs promoter having a sequence of nucleotides No. 380...625 of SEQ ID NO: 4.

7. Adenovirus according to one of claims 1 to 6, **characterized in that** the first promoter is a CMVgal promoter having a sequence of nucleotides No. 321..963 of SEQ ID NO: 6, and wherein the DNA comprises an expression cassette encoding GAL4-KRAB having a sequence of nucleotides No. 6539..7144 of SEQ ID NO: 6 under the control of a promoter that is activated by the presence of p53.

8. Adenovirus according to one of the preceding claims, **characterized in that** the E1A encoding DNA sequence is devoid of the coding DNA sequence encoding amino acids No. 4 to 25 of the wild-type E1A amino acid sequence.

9. Adenovirus according to one of the preceding claims, **characterized in that** the promoter that is activated by the presence of p53 is the prMinRGC promoter having a nucleotide sequence of No. 5872 to 6230 of SEQ ID NO: 4.

10. Adenovirus according to one of claims 3 to 8, **characterized in that** the expression cassette encoding an effector molecule encodes at least one cytokine.

11. Adenovirus according to one of claims 3 to 9, **characterized in that** the expression cassette encoding an effector molecule encodes a fusion protein comprising at least two of or the three of FLT3L and MIP1a and XCL1, and a 2A element between each two of these.

12. Adenovirus according to one of the preceding claims for use in the treatment of a tumour that has p53 at a reduced activity level.

13. Adenovirus for use in the treatment of a tumour that has p53 at a reduced activity level according to claim 12, for inducing a tumour-specific T-cell immune response against the tumour.

14. Adenovirus according to one of the preceding claims for use in the treatment of a tumour according to one of claims 12 to 13, **characterized by** the use being intra-tumoral administration.

15. Process for producing adenovirus in cultivated mammalian cells, **characterized by** the adenovirus being one according to one of the preceding claims.


SEQUENCE LISTING

<110> Medizinische Hochschule Hannover

<120> Adenovirus for anti-tumour therapy

<130> M1090EP

<160> 6

<170> BiSSAP 1.3.6

<210> 1
<211> 114
<212> PRT
<213> Artificial Sequence

<220>
<223> E4 orf4-encoded protein

<400> 1
Met Val Leu Pro Ser Leu Pro Ser Pro Leu Leu Leu Glu Thr Gln Ser
1               5                   10                  15
Ser Cys Ile Ala Trp Leu Gly Phe Ala Tyr Ala Thr Val Asp Asp Phe
            20                  25                  30
Leu Arg Thr Ile Arg Cys Asp Gly Val Leu Ile Thr Thr Glu Ala Ser
        35                  40                  45
Ile Leu Leu Thr Asn Leu Arg Val Trp Leu Tyr Leu Asn Tyr His Thr
    50                  55                  60
Glu His Ala Lys Arg Gln Asp Arg Arg Arg Arg Ser Val Cys His Ala
65                  70                  75                  80
Arg Thr Trp Phe Cys Phe Arg Lys Tyr Asp Tyr Val Arg Arg Ser Ile
                85                  90                  95
Trp His Asp Thr Thr Thr Asn Thr Ile Ser Val Val Ser Ala His Ser
            100                 105                 110
Val Gln

<210> 2
<211> 114
<212> PRT
<213> Artificial Sequence

<220>
<223> E4 orf4-encoded protein

<400> 2
Met Ile Leu Pro Ser Leu Pro Ser Pro Leu Leu Leu Glu Thr Gln Ser
1               5                   10                  15
Ser Cys Ile Ala Trp Leu Gly Phe Ala Tyr Ala Thr Val Asp Asp Phe
            20                  25                  30
Leu Arg Thr Ile Lys Tyr Asp Glu Val Leu Ile Thr Thr Glu Ala Ser
        35                  40                  45
Ile Leu Leu Thr Asn Leu Arg Val Trp Leu Tyr Leu Asn Tyr Gln Thr
    50                  55                  60
Glu His Thr Lys Arg Gln Asp Arg Arg Arg Arg Ser Val Cys His Ala
65                  70                  75                  80
Arg Thr Trp Phe Cys Phe Arg Lys Tyr Asp Tyr Val Arg Arg Ser Ile
                85                  90                  95
Trp His Asp Thr Thr Thr Asn Thr Ile Ser Val Val Ser Ala His Ser
            100                 105                 110

Val Gln

<210> 3
<211> 114
<212> PRT
<213> Artificial Sequence

<220>
<223> E4 orf4-encoded protein

<400> 3
Met Ile Leu Pro Ser Leu Pro Ser Pro Leu Leu Leu Glu Thr Gln Ser
1               5                   10                  15
Ser Cys Ile Ala Trp Leu Gly Phe Ala Tyr Ala Thr Val Asp Asp Phe
            20                  25                  30
Leu Arg Thr Ile Lys Tyr Asp Gly Val Leu Ile Thr Thr Glu Ala Ser
        35                  40                  45
Ile Leu Leu Thr Asn Leu Arg Val Trp Leu Tyr Leu Asn Tyr Gln Thr
    50                  55                  60
Glu His Thr Lys Arg Gln Asp Arg Arg Arg Arg Ser Val Cys His Ala
65                  70                  75                  80
Arg Thr Trp Phe Cys Phe Arg Lys Tyr Asp Tyr Val Arg Arg Ser Ile
                85                  90                  95
Trp His Asp Thr Thr Thr Asn Thr Ile Ser Val Val Ser Ala His Ser
            100                 105                 110
Val Gln

<210> 4
<211> 37021
<212> DNA
<213> Artificial Sequence

<220>
<223> Ad5 including E4 orf4-encoded protein

<220>
<221> LTR
<222> 1..103
<223> /note="L-ITR"

<220>
<221> promoter
<222> 380..625
<223> /note="CMVs promoter"

<220>
<221> gene
<222> 727..1646
<223> /gene="E1A gene, codons for amino acids 4 to 25 of wild-type
      deleted"

<220>
<221> misc_feature
<222> 837..857
<223> /note="RNAi target site"

<220>
<221> misc_feature
<222> 886..909

<223> /note="RNAi target site"

<220>
<221> misc_feature
<222> 1684..1764
<223> /note="heterologous target-sites for RNAi inserted into the 3UTR
      of E1A, equivalent to E1A-886..909, E1A-837..857, Prot
      IX7353..7374)"

<220>
<221> promoter
<222> 1813..1910
<223> /note="promoter for E1B"

<220>
<221> gene
<222> 1911..2441
<223> /gene="E1B-19k"

<220>
<221> gene
<222> 2216..3706
<223> /gene="E1B-55k"

<220>
<221> protein_bind
<222> 3731..4320
<223> /note="IRES"

<220>
<221> CDS
<222> 4349..5053
<223> /note="FLT3L"
      /transl_table=1

<220>
<221> misc_feature
<222> 5054..5116
<223> /note="2A"

<220>
<221> gene
<222> 5117..5392
<223> /gene="MIP1A/CCL3"

<220>
<221> misc_feature
<222> 5393..5455
<223> /note="2A"

<220>
<221> gene
<222> 5456..5800
<223> /gene="XCL1"

<220>
<221> promoter
<222> 5872..6230
<223> /note="prMinRGC"

<220>
<221> misc_feature
<222> 6259..6321

<223> /note="inhibitory shRNA for E1A (1)"

<220>
<221> misc_feature
<222> 6259..7064
<223> /note="shRNA network"

<220>
<221> misc_feature
<222> 6387..6444
<223> /note="inhibitory shRNA for E1A (2)"

<220>
<221> misc_feature
<222> 6511..6567
<223> /note="inhibitory shRNA for Portein IX"

<220>
<221> misc_feature
<222> 6634..6690
<223> /note="inhibitory shRNA for E4 orf6 (1)"

<220>
<221> misc_feature
<222> 6756..6816
<223> /note="inhibitory shRNA for E4 orf6 (2)"

<220>
<221> misc_feature
<222> 6882..6939
<223> /note="inhibitory shRNA for TP"

<220>
<221> misc_feature
<222> 7006..7064
<223> /note="inhibitory shRNA for AdPol"

<220>
<221> gene
<222> 7332..7754
<223> /note="Protein IX, part of backbone"

<220>
<221> gene
<222> 8920..17842
<223> /note="polymerase, Ad-Pol, part of backbone"

<220>
<221> gene
<222> 12306..17842
<223> /gene="terminal protein, TP, part of backbone"

<220>
<221> gene
<222> 17878..19593
<223> /gene="Protein III: Penton, part of backbone"

<220>
<221> gene
<222> 22563..25421
<223> /gene="hexon, part of backbone"

<220>

<221> gene
<222> 26164..27753
<223> /gene="reverse complementary sequence of DNA-binding protein,
      dbd, part of backbone"

<220>
<221> gene
<222> 27782..30205
<223> /gene="100k, part of backbone"

<220>
<221> misc_feature
<222> 31589
<223> /note="deltaE3, (equivalent to del. of nt 28137  30818 in the
      wildtype virus)"

<220>
<221> gene
<222> 32108..33853
<223> /gene="Fiber, part of backbone"

<220>
<221> gene
<222> 33980..35143
<223> /gene="reverse complementary sequence of E4 orf6/7"

<220>
<221> gene
<222> 35064..35408
<223> /gene="reverse complementary sequence of hybrid E4 orf4 Ad5/Ad11,
      amino acid sequence is SEQ ID NO: 1"

<220>
<221> misc_feature
<222> 35419..35770
<223> /note="reverse complementary sequence of inactivated E4 orf3
      locus"

<220>
<221> terminator
<222> 35747..35749
<223> /note="stop codon E4orf3"

<220>
<221> gene
<222> 35782..36175
<223> /gene="reverse complementary sequence of E4orf2"

<220>
<221> gene
<222> 36207..36593
<223> /gene="reverse complementary sequence of E4orf1"

<220>
<221> LTR
<222> 36919..37021
<223> /note="R-ITR"

<400> 4
catcatcaat aatatacctt attttggatt gaagccaata tgataatgag ggggtggagt      60

ttgtgacgtg gcgcggggcg tgggaacggg gcgggtgacg tagtagtgtg gcggaagtgt     120

gatgttgcaa gtgtggcgga acacatgtaa gcgacggatg tggcaaaagt gacgtttttg 180

gtgtgcgccg gtgtacacag gaagtgacaa ttttcgcgcg gttttaggcg gatgttgtag 240

taaatttggg cgtaaccgag taagatttgg ccattttcgc gggaaaactg aataagagga 300

agtgaaatct gaataatttt gtgttactca tagcgcgtaa tctctagcat cgtaactata 360

acggtcctaa ggtagcgaaa gctagtacca tggtgatgcg gttttggcag tacatcaatg 420

gcggtggata gcggtccgac tcacggggat ttccaagtct ccaccccatt gacgtcaatg 480

ggagtttgtt ttggcaccaa aatcaacggg actttccaaa atgtcgtaac aactccgccc 540

cacggacgca aatgggccgt aggcgtgtac ggtgggcggt ctatataagc ccggctggtt 600

tagtgaaccg tcagatcctc gagggcgtgt agtgtattta tacccggtga gttcctcaag 660

aggccactct tgagtgccag cgagtagagt tttctcctcc gagccgctcc gacaccggga 720

ctgaaaatga gacatgaggt actggctgat aatcttccac ctcctagcca ttttgaacca 780

cctacccttc acgaactgta tgatttagac gtgacggccc ccgaagatcc caacgaggag 840

gcggtttcgc agatttttcc cgactctgta atgttggcgg tgcaggaagg gattgactta 900

ctcacttttc cgccggcgcc cggttctccg gagccgcctc acctttcccg gcagcccgag 960

cagccggagc agagagcctt gggtccggtt tctatgccaa accttgtacc ggaggtgatc 1020

gatcttacct gccacgaggc tggctttcca cccagtgacg acgaggatga agagggtgag 1080

gagtttgtgt tagattatgt ggagcacccc gggcacggtt gcaggtcttg tcattatcac 1140

cggaggaata cgggggaccc agatattatg tgttcgcttt gctatatgag gacctgtggc 1200

atgtttgtct acagtaagtg aaaattatgg gcagtgggtg atagagtggt gggtttggtg 1260

tggtaatttt ttttttaatt tttacagttt tgtggtttaa agaattttgt attgtgattt 1320

ttttaaaagg tcctgtgtct gaacctgagc ctgagcccga gccagaaccg gagcctgcaa 1380

gacctacccg ccgtcctaaa atggcgcctg ctatcctgag acgcccgaca tcacctgtgt 1440

ctagagaatg caatagtagt acggatagct gtgactccgg tccttctaac acacctcctg 1500

agatacaccc ggtggtcccg ctgtgcccca ttaaaccagt tgccgtgaga gttggtgggc 1560

gtcgccaggc tgtggaatgt atcgaggact tgcttaacga gcctgggcaa cctttggact 1620

tgagctgtaa acgccccagg ccataaggtg taaacctgtg attgcgtgtg tggttaacac 1680

acaggaggcg gtttcgcaga tttttacaca cagaagggat tgacttactc acttttacac 1740

acaggaagca ttgtgagctc atatgagacc caacgccttt gtttgctgaa tgagttgatg 1800

taagtttaat aaagggtgag ataatgttta acttgcatgg cgtgttaaat ggggcggggc 1860

ttaaagggta tataatgcgc cgtgggctaa tcttggttac atctgacctc atggaggctt 1920

gggagtgttt ggaagatttt tctgctgtgc gtaacttgct ggaacagagc tctaacagta 1980

cctcttggtt ttggaggttt ctgtggggct catcccaggc aaagttagtc tgcagaatta 2040

aggaggatta caagtgggaa tttgaagagc ttttgaaatc ctgtggtgag ctgtttgatt 2100

ctttgaatct gggtcaccag gcgcttttcc aagagaaggt catcaagact ttggattttt 2160

ccacaccggg gcgcgctgcg gctgctgttg cttttttgag ttttataaag gataaatgga 2220

gcgaagaaac ccatctgagc ggggggtacc tgctggattt tctggccatg catctgtgga 2280

gagcggttgt gagacacaag aatcgcctgc tactgttgtc ttccgtccgc ccggcgataa 2340

taccgacgga ggagcagcag cagcagcagg aggaagccag gcggcggcgg caggagcaga 2400

gcccatggaa cccgagagcc ggcctggacc ctcgggaatg aatgttgtac aggtggctga 2460

actgtatcca gaactgagac gcattttgac aattacagag gatgggcagg ggctaaaggg 2520

ggtaaagagg gagcgggggg cttgtgaggc tacagaggag gctaggaatc tagcttttag 2580

cttaatgacc agacaccgtc ctgagtgtat tacttttcaa cagatcaagg ataattgcgc 2640

taatgagctt gatctgctgg cgcagaagta ttccatagag cagctgacca cttactggct 2700

gcagccaggg gatgattttg aggaggctat tagggtatat gcaaaggtgg cacttaggcc 2760

agattgcaag tacaagatca gcaaacttgt aaatatcagg aattgttgct acatttctgg 2820

gaacggggcc gaggtggaga tagatacgga ggatagggtg gcctttagat gtagcatgat 2880

aaatatgtgg ccgggggtgc ttggcatgga cggggtggtt attatgaatg taaggtttac 2940

tggccccaat tttagcggta cggttttcct ggccaatacc aaccttatcc tacacggtgt 3000

aagcttctat gggtttaaca atacctgtgt ggaagcctgg accgatgtaa gggttcgggg 3060

ctgtgccttt tactgctgct ggaagggggt ggtgtgtcgc cccaaaagca gggcttcaat 3120

taagaaatgc ctctttgaaa ggtgtacctt gggtatcctg tctgagggta actccagggt 3180

gcgccacaat gtggcctccg actgtggttg cttcatgcta gtgaaaagcg tggctgtgat 3240

taagcataac atggtatgtg gcaactgcga ggacagggcc tctcagatgc tgacctgctc 3300

ggacggcaac tgtcacctgc tgaagaccat tcacgtagcc agccactctc gcaaggcctg 3360

gccagtgttt gagcataaca tactgacccg ctgttccttg catttgggta acaggagggg 3420

ggtgttccta ccttaccaat gcaatttgag tcacactaag atattgcttg agcccgagag 3480

catgtccaag gtgaacctga acggggtgtt tgacatgacc atgaagatct ggaaggtgct 3540

gaggtacgat gagacccgca ccaggtgcag accctgcgag tgtggcggta aacatattag 3600

gaaccagcct gtgatgctgg atgtgaccga ggagctgagg cccgatcact tggtgctggc 3660

ctgcacccgc gctgagtttg gctctagcga tgaagataca gattgaggta ctgaaatgtg 3720

tgggcgtggc gaattccgcc cctctccctc ccccccccct aacgttactg gccgaagccg 3780

cttggaataa ggccggtgtg cgtttgtcta tatgttattt tccaccatat tgccgtcttt 3840

tggcaatgtg agggcccgga aacctggccc tgtcttcttg acgagcattc ctaggggtct 3900

ttcccctctc gccaaaggaa tgcaaggtct gttgaatgtc gtgaaggaag cagttcctct 3960

ggaagcttct tgaagacaaa caacgtctgt agcgaccctt tgcaggcagc ggaacccccc 4020

acctggcgac aggtgcctct gcggccaaaa gccacgtgta taagatacac ctgcaaaggc 4080

ggcacaaccc cagtgccacg ttgtgagttg gatagttgtg gaaagagtca aatggctctc 4140

ctcaagcgta ttcaacaagg ggctgaagga tgcccagaag gtaccccatt gtatgggatc 4200

tgatctgggg cctcggtgca catgctttac atgtgtttag tcgaggttaa aaaaacgtct 4260

aggccccccg aaccacgggg acgtggtttt cctttgaaaa acacgatgat aagcttgatc 4320

acgcgtttcg aacgatttaa atttaaat atg aca gtg ctg gcg cca gcc tgg 4372
Met Thr Val Leu Ala Pro Ala Trp
1               5

agc cca aca acc tat ctc ctc ctg ctg ctg ctg ctg agc tcg gga ctc 4420
Ser Pro Thr Thr Tyr Leu Leu Leu Leu Leu Leu Leu Ser Ser Gly Leu
    10                  15                  20

agt ggg acc cag gac tgc tcc ttc caa cac agc ccc atc tcc tcc gac 4468
Ser Gly Thr Gln Asp Cys Ser Phe Gln His Ser Pro Ile Ser Ser Asp
25                  30                  35                  40

ttc gct gtc aaa atc cgt gag ctg tct gac tac ctg ctt caa gat tac 4516
Phe Ala Val Lys Ile Arg Glu Leu Ser Asp Tyr Leu Leu Gln Asp Tyr
                45                  50                  55

cca gtc acc gtg gcc tcc aac ctg cag gac gag gag ctc tgc ggg ggc 4564
Pro Val Thr Val Ala Ser Asn Leu Gln Asp Glu Glu Leu Cys Gly Gly
            60                  65                  70

ctc tgg cgg ctg gtc ctg gca cag cgc tgg atg gag cgg ctc aag act 4612
Leu Trp Arg Leu Val Leu Ala Gln Arg Trp Met Glu Arg Leu Lys Thr
        75                  80                  85

gtc gct ggg tcc aag atg caa ggc ttg ctg gag cgc gtg aac acg gag 4660
Val Ala Gly Ser Lys Met Gln Gly Leu Leu Glu Arg Val Asn Thr Glu
    90                  95                  100

ata cac ttt gtc acc aaa tgt gcc ttt cag ccc ccc ccc agc tgt ctt 4708
Ile His Phe Val Thr Lys Cys Ala Phe Gln Pro Pro Pro Ser Cys Leu
105                 110                 115                 120

cgc ttc gtc cag acc aac atc tcc cgc ctc ctg cag gag acc tcc gag 4756
Arg Phe Val Gln Thr Asn Ile Ser Arg Leu Leu Gln Glu Thr Ser Glu
                125                 130                 135

cag ctg gtg gcg ctg aag ccc tgg atc act cgc cag aac ttc tcc cgg 4804
Gln Leu Val Ala Leu Lys Pro Trp Ile Thr Arg Gln Asn Phe Ser Arg
            140                 145                 150

tgc ctg gag ctg cag tgt cag ccc gac tcc tca acc ctg cca ccc cca 4852
Cys Leu Glu Leu Gln Cys Gln Pro Asp Ser Ser Thr Leu Pro Pro Pro
        155                 160                 165

tgg agt ccc cgg ccc ctg gag gcc aca gcc ccg aca gcc ccg cag ccc 4900
Trp Ser Pro Arg Pro Leu Glu Ala Thr Ala Pro Thr Ala Pro Gln Pro
    170                 175                 180

cct ctg ctc ctc cta ctg ctg ctg ccc gtg ggc ctc ctg ctg ctg gct 4948

Pro Leu Leu Leu Leu Leu Leu Leu Pro Val Gly Leu Leu Leu Leu Ala
185                 190                 195                 200

gct gcc tgg tgc ctg cac tgg cag agg acg cgg cgg agg aca ccc cgc     4996
Ala Ala Trp Cys Leu His Trp Gln Arg Thr Arg Arg Arg Thr Pro Arg
                205                 210                 215

cct ggg gag cag gtg ccc ccc gtc ccc agt ccc cag gac ctg ctg ctt     5044
Pro Gly Glu Gln Val Pro Pro Val Pro Ser Pro Gln Asp Leu Leu Leu
            220                 225                 230

gtg gag cac tccggagcca cgaacttctc tctgttaaag caagcaggag             5093
Val Glu His
        235

acgtggaaga aaaccccggt cccatgcagg tctccactgc tgcccttgct gtcctcctct   5153

gcaccatggc tctctgcaac cagttctctg catcacttgc tgctgacacg ccgaccgcct   5213

gctgcttcag ctacacctcc cggcagattc cacagaattt catagctgac tactttgaga   5273

cgagcagcca gtgctccaag cccggtgtca tcttcctaac caagcgaagc cggcaggtct   5333

gtgctgaccc cagtgaggag tgggtccaga aatatgtcag cgacctggag ctgagtgcct   5393

ccggagccac gaacttctct ctgttaaagc aagcaggaga cgtggaagaa aaccccggtc   5453

ccatgagact tctcatcctg gccctccttg gcatctgctc tctcactgca tacattgtgg   5513

aaggtgtagg gagtgaagtc tcagataaga ggacctgtgt gagcctcact acccagcgac   5573

tgccagttag cagaatcaag acctacacca tcacggaagg ctccttgaga gcagtaattt   5633

ttattaccaa acgtggccta aaagtctgtg ctgatccaca agccacatgg gtgagagacg   5693

tggtcaggag catggacagg aaatccaaca ccagaaataa catgatccag accaagccaa   5753

caggaaccca gcaatcgacc aatacagctg tgactctgac tggctagctc gagaataaag   5813

gtaccttatt ttcattagat ttgtgtgttg gttttttgtg tggcggcctc gagatgggag   5873

agcttgatgc caggcaagtc caggcaggcc aggcaagtcc aggcaggcca ggcaagtcca   5933

ggcaggccag gcaagtccag gcaggccagg caagtccagg caggccaggc aagtccaggc   5993

aggccaggca agtccaggca ggccaggcaa gtccaggcag gccaggcaag tccaggcagg   6053

ccaggcaagt ccaggcaggg ccaggcaagt ccaggcaggc caggcaagtc caggcaggcc   6113

aggcaagtcc aggcaggatc gaattcgagc tcgtacggtg ggaggcctat ataagcagac   6173

ctcgtttagt gaaccgtcag atcgcctgga gacgaagctt gctagcggat cctcgagaag   6233

gtatattgct gttgacagtg agcgagaagg gattgactta ctcacttttg tgaagccaca   6293

gatgaaaagt gagtaagtca atcccttcgt gcctactgcc tcggaattca ctagcggatc   6353

ctcgagaagg tatattgctg ttgacagtga gcgaggaggc ggtttcgcag attttgtgaa   6413

gccacagatg aaaatctgcg aaaccgcctc cgtgcctact gcctcggaat tcactagcgg   6473

atcctcgaga aggtatattg ctgttgacag tgagcgagga agcattgtga gctcatatgt   6533

gaagccacag atgatatgag ctcacaatgc ttccgtgcct actgcctcgg aattcactag 6593

cggatcctcg agaaggtata ttgctgttga cagtgagcga gacaggaaac cgtgtggaat 6653

agtgaagcca cagatgtatt ccacacggtt tcctgtcgtg cctactgcct cggaattcac 6713

tagcggatcc tcgaaaggta tattgctgtt gacagtgagc gaccatgttt aatcagaggt 6773

ttatagtgaa gccacagatg tataaacctc tgattaaaca tgggtgccta ctgcctcgga 6833

attcactagc ggatcctcga gaaggtatat tgctgttgac agtgagcgac ggagtaccta 6893

cacaacaatt gtgaagccac agatgaattg ttgtgtaggt actccggtgc ctactgcctc 6953

ggaattcact agcggatcct cgagaaggta tattgctgtt gacagtgagc gagcgaatac 7013

gtgcagctaa acatgtgaag ccacagatga tgtttagctg cacgtattcg cgtgcctact 7073

gcctcggaat tcactagcta gtcatatgct gcagtgtaga tctaaataaa gcaatagcat 7133

cacaaataaa gcatttcatt cactgcattc tagttgtggt ttgtacgcgt gatatcaatt 7193

catctatgtc gggtgcggag aaagaggtaa tgaaatggca tcgactcgaa gatctgggcg 7253

tggttaaggg tgggaaagaa tatataaggt gggggtctta tgtagttttg tatctgtttt 7313

gcagcagccg ccgccgccat gagcaccaac tcgtttgatg gaagcattgt gagctcatat 7373

ttgacaacgc gcatgccccc atgggccggg gtgcgtcaga atgtgatggg ctccagcatt 7433

gatggtcgcc ccgtcctgcc cgcaaactct actaccttga cctacgagac cgtgtctgga 7493

acgccgttgg agactgcagc ctccgccgcc gcttcagccg ctgcagccac cgcccgcggg 7553

attgtgactg actttgcttt cctgagcccg cttgcaagca gtgcagcttc ccgttcatcc 7613

gcccgcgatg acaagttgac ggctcttttg gcacaattgg attctttgac ccgggaactt 7673

aatgtcgttt ctcagcagct gttggatctg cgccagcagg tttctgccct gaaggcttcc 7733

tcccctccca atgcggttta aaacataaat aaaaaaccag actctgtttg gatttggatc 7793

aagcaagtgt cttgctgtct ttatttaggg gttttgcgcg cgcggtaggc ccgggaccag 7853

cggtctcggt cgttgagggt cctgtgtatt ttttccagga cgtggtaaag gtgactctgg 7913

atgttcagat acatgggcat aagcccgtct ctggggtgga ggtagcacca ctgcagagct 7973

tcatgctgcg gggtggtgtt gtagatgatc cagtcgtagc aggagcgctg ggcgtggtgc 8033

ctaaaaatgt ctttcagtag caagctgatt gccaggggca ggcccttggt gtaagtgttt 8093

acaaagcggt taagctggga tgggtgcata cgtggggata tgagatgcat cttggactgt 8153

atttttaggt tggctatgtt cccagccata tccctccggg gattcatgtt gtgcagaacc 8213

accagcacag tgtatccggt gcacttggga aatttgtcat gtagcttaga aggaaatgcg 8273

tggaagaact tggagacgcc cttgtgacct ccaagatttt ccatgcattc gtccataatg 8333

atggcaatgg gcccacgggc ggcggcctgg gcgaagatat ttctgggatc actaacgtca 8393

tagttgtgtt ccaggatgag atcgtcatag gccattttta caaagcgcgg gcggagggtg 8453

ccagactgcg gtataatggt tccatccggc ccaggggcgt agttaccctc acagatttgc 8513

atttcccacg ctttgagttc agatgggggg atcatgtcta cctgcggggc gatgaagaaa 8573

acggtttccg ggtaggggga gatcagctgg gaagaaagca ggttcctgag cagctgcgac 8633

ttaccgcagc cggtgggccc gtaaatcaca cctattaccg gctgcaactg gtagttaaga 8693

gagctgcagc tgccgtcatc cctgagcagg ggggccactt cgttaagcat gtccctgact 8753

cgcatgtttt ccctgaccaa atccgccaga aggcgctcgc cgcccagcga tagcagttct 8813

tgcaaggaag caaagttttt caacggtttg agaccgtccg ccgtaggcat gcttttgagc 8873

gtttgaccaa gcagttccag gcggtcccac agctcggtca cctgctctac ggcatctcga 8933

tccagcatat ctcctcgttt cgcgggttgg ggcggctttc gctgtacggc agtagtcggt 8993

gctcgtccag acgggccagg gtcatgtctt tccacgggcg cagggtcctc gtcagcgtag 9053

tctgggtcac ggtgaagggg tgcgctccgg gctgcgcgct ggccagggtg cgcttgaggc 9113

tggtcctgct ggtgctgaag cgctgccggt cttcgccctg cgcgtcggcc aggtagcatt 9173

tgaccatggt gtcatagtcc agcccctccg cggcgtggcc cttggcgcgc agcttgccct 9233

tggaggaggc gccgcacgag gggcagtgca gacttttgag ggcgtagagc ttgggcgcga 9293

gaaataccga ttccggggag taggcatccg cgccgcaggc cccgcagacg gtctcgcatt 9353

ccacgagcca ggtgagctct ggccgttcgg ggtcaaaaac caggtttccc ccatgctttt 9413

tgatgcgttt cttacctctg gtttccatga gccggtgtcc acgctcggtg acgaaaaggc 9473

tgtccgtgtc cccgtataca gacttgagag gcctgtcctc gagcggtgtt ccgcggtcct 9533

cctcgtatag aaactcggac cactctgaga caaaggctcg cgtccaggcc agcacgaagg 9593

aggctaagtg ggaggggtag cggtcgttgt ccactagggg gtccactcgc tccagggtgt 9653

gaagacacat gtcgccctct tcggcatcaa ggaaggtgat tggtttgtag gtgtaggcca 9713

cgtgaccggg tgttcctgaa ggggggctat aaaagggggt gggggcgcgt tcgtcctcac 9773

tctcttccgc atcgctgtct gcgagggcca gctgttgggg tgagtactcc ctctgaaaag 9833

cgggcatgac ttctgcgcta agattgtcag tttccaaaaa cgaggaggat ttgatattca 9893

cctggcccgc ggtgatgcct ttgagggtgg ccgcatccat ctggtcagaa aagacaatct 9953

ttttgttgtc aagcttggtg gcaaacgacc cgtagagggc gttggacagc aacttggcga 10013

tggagcgcag ggtttggttt ttgtcgcgat cggcgcgctc cttggccgcg atgtttagct 10073

gcacgtattc gcgcgcaacg caccgccatt cgggaaagac ggtggtgcgc tcgtcgggca 10133

ccaggtgcac gcgccaaccg cggttgtgca gggtgacaag gtcaacgctg gtggctacct 10193

ctccgcgtag gcgctcgttg gtccagcaga ggcggccgcc cttgcgcgag cagaatggcg 10253

gtagggggtc tagctgcgtc tcgtccgggg ggtctgcgtc cacggtaaag accccgggca 10313

gcaggcgcgc gtcgaagtag tctatcttgc atccttgcaa gtctagcgcc tgctgccatg 10373

cgcgggcggc aagcgcgcgc tcgtatgggt tgagtggggg accccatggc atggggtggg 10433

tgagcgcgga ggcgtacatg ccgcaaatgt cgtaaacgta gaggggctct ctgagtattc 10493

caagatatgt agggtagcat cttccaccgc ggatgctggc gcgcacgtaa tcgtatagtt 10553

cgtgcgaggg agcgaggagg tcgggaccga ggttgctacg ggcgggctgc tctgctcgga 10613

agactatctg cctgaagatg gcatgtgagt tggatgatat ggttggacgc tggaagacgt 10673

tgaagctggc gtctgtgaga cctaccgcgt cacgcacgaa ggaggcgtag gagtcgcgca 10733

gcttgttgac cagctcggcg gtgacctgca cgtctagggc gcagtagtcc agggtttcct 10793

tgatgatgtc atacttatcc tgtccctttt ttttccacag ctcgcggttg aggacaaact 10853

cttcgcggtc tttccagtac tcttggatcg gaaacccgtc ggcctccgaa cggtaagagc 10913

ctagcatgta gaactggttg acggcctggt aggcgcagca tcccttttct acgggtagcg 10973

cgtatgcctg cgcggccttc cggagcgagg tgtgggtgag cgcaaaggtg tccctgacca 11033

tgactttgag gtactggtat ttgaagtcag tgtcgtcgca tccgccctgc tcccagagca 11093

aaaagtccgt gcgctttttg gaacgcggat ttggcagggc gaaggtgaca tcgttgaaga 11153

gtatctttcc cgcgcgaggc ataaagttgc gtgtgatgcg gaagggtccc ggcacctcgg 11213

aacggttgtt aattacctgg gcggcgagca cgatctcgtc aaagccgttg atgttgtggc 11273

ccacaatgta aagttccaag aagcgcggga tgcccttgat ggaaggcaat tttttaagtt 11333

cctcgtaggt gagctcttca ggggagctga gcccgtgctc tgaaagggcc cagtctgcaa 11393

gatgagggtt ggaagcgacg aatgagctcc acaggtcacg ggccattagc atttgcaggt 11453

ggtcgcgaaa ggtcctaaac tggcgaccta tggccatttt ttctggggtg atgcagtaga 11513

aggtaagcgg gtcttgttcc cagcggtccc atccaaggtt cgcggctagg tctcgcgcgg 11573

cagtcactag aggctcatct ccgccgaact tcatgaccag catgaagggc acgagctgct 11633

tcccaaaggc ccccatccaa gtataggtct ctacatcgta ggtgacaaag agacgctcgg 11693

tgcgaggatg cgagccgatc gggaagaact ggatctcccg ccaccaattg gaggagtggc 11753

tattgatgtg gtgaaagtag aagtccctgc gacgggccga acactcgtgc tggcttttgt 11813

aaaaacgtgc gcagtactgg cagcggtgca cgggctgtac atcctgcacg aggttgacct 11873

gacgaccgcg cacaaggaag cagagtggga atttgagccc ctcgcctggc gggtttggct 11933

ggtggtcttc tacttcggct gcttgtcctt gaccgtctgg ctgctcgagg ggagttacgg 11993

tggatcggac caccacgccg cgcgagccca aagtccagat gtccgcgcgc ggcggtcgga 12053

gcttgatgac aacatcgcgc agatgggagc tgtccatggt ctggagctcc cgcggcgtca 12113

ggtcaggcgg gagctcctgc aggtttacct cgcatagacg ggtcagggcg cgggctagat 12173

ccaggtgata cctaatttcc aggggctggt tggtggcggc gtcgatggct tgcaagaggc 12233

cgcatccccg cggcgcgact acggtaccgc gcggcgggcg gtgggccgcg ggggtgtcct 12293

tggatgatgc atctaaaagc ggtgacgcgg gcgagccccc ggaggtaggg ggggctccgg 12353

acccgccggg agagggggca ggggcacgtc ggcgccgcgc gcgggcagga gctggtgctg 12413

cgcgcgtagg ttgctggcga acgcgacgac gcggcggttg atctcctgaa tctggcgcct 12473

ctgcgtgaag acgacgggcc cggtgagctt gagcctgaaa gagagttcga cagaatcaat 12533

ttcggtgtcg ttgacggcgg cctggcgcaa aatctcctgc acgtctcctg agttgtcttg 12593

ataggcgatc tcggccatga actgctcgat ctcttcctcc tggagatctc cgcgtccggc 12653

tcgctccacg gtggcggcga ggtcgttgga aatgcgggcc atgagctgcg agaaggcgtt 12713

gaggcctccc tcgttccaga cgcggctgta gaccacgccc ccttcggcat cgcgggcgcg 12773

catgaccacc tgcgcgagat tgagctccac gtgccgggcg aagacggcgt agtttcgcag 12833

gcgctgaaag aggtagttga gggtggtggc ggtgtgttct gccacgaaga agtacataac 12893

ccagcgtcgc aacgtggatt cgttgatatc ccccaaggcc tcaaggcgct ccatggcctc 12953

gtagaagtcc acggcgaagt tgaaaaactg ggagttgcgc gccgacacgg ttaactcctc 13013

ctccagaaga cggatgagct cggcgacagt gtcgcgcacc tcgcgctcaa aggctacagg 13073

ggcctcttct tcttcttcaa tctcctcttc cataagggcc tccccttctt cttcttctgg 13133

cggcggtggg ggagggggga cacggcggcg acgacggcgc accgggaggc ggtcgacaaa 13193

gcgctcgatc atctccccgc ggcgacggcg catggtctcg gtgacggcgc ggccgttctc 13253

gcgggggcgc agttggaaga cgccgcccgt catgtcccgg ttatgggttg gcggggggct 13313

gccatgcggc agggatacgg cgctaacgat gcatctcaac aattgttgtg taggtactcc 13373

gccgccgagg gacctgagcg agtccgcatc gaccggatcg gaaaacctct cgagaaaggc 13433

gtctaaccag tcacagtcgc aaggtaggct gagcaccgtg gcgggcggca gcgggcggcg 13493

gtcggggttg tttctggcgg aggtgctgct gatgatgtaa ttaaagtagg cggtcttgag 13553

acggcggatg gtcgacagaa gcaccatgtc cttgggtccg gcctgctgaa tgcgcaggcg 13613

gtcggccatg ccccaggctt cgttttgaca tcggcgcagg tctttgtagt agtcttgcat 13673

gagcctttct accggcactt cttcttctcc ttcctcttgt cctgcatctc ttgcatctat 13733

cgctgcggcg gcggcggagt ttggccgtag gtggcgccct cttcctccca tgcgtgtgac 13793

cccgaagccc ctcatcggct gaagcagggc taggtcggcg acaacgcgct cggctaatat 13853

ggcctgctgc acctgcgtga gggtagactg gaagtcatcc atgtccacaa agcggtggta 13913

tgcgcccgtg ttgatggtgt aagtgcagtt ggccataacg gaccagttaa cggtctggtg 13973

acccggctgc gagagctcgg tgtacctgag acgcgagtaa gccctcgagt caaatacgta 14033

gtcgttgcaa gtccgcacca ggtactggta tcccaccaaa aagtgcggcg gcggctggcg 14093

gtagaggggc cagcgtaggg tggccggggc tccgggggcg agatcttcca acataaggcg 14153
atgatatccg tagatgtacc tggacatcca ggtgatgccg gcggcggtgg tggaggcgcg 14213
cggaaagtcg cggacgcggt tccagatgtt gcgcagcggc aaaaagtgct ccatggtcgg 14273
gacgctctgg ccggtcaggc gcgcgcaatc gttgacgctc tagcgtgcaa aaggagagcc 14333
tgtaagcggg cactcttccg tggtctggtg gataaattcg caagggtatc atggcggacg 14393
accggggttc gagccccgta tccggccgtc cgccgtgatc catgcggtta ccgcccgcgt 14453
gtcgaaccca ggtgtgcgac gtcagacaac gggggagtgc tccttttggc ttccttccag 14513
gcgcggcggc tgctgcgcta gcttttttgg ccactggccg cgcgcagcgt aagcggttag 14573
gctggaaagc gaaagcatta agtggctcgc tccctgtagc cggagggtta ttttccaagg 14633
gttgagtcgc gggacccccg gttcgagtct cggaccggcc ggactgcggc gaacgggggt 14693
ttgcctcccc gtcatgcaag accccgcttg caaattcctc cggaaacagg gacgagcccc 14753
ttttttgctt ttcccagatg catccggtgc tgcggcagat gcgcccccct cctcagcagc 14813
ggcaagagca agagcagcgg cagacatgca gggcaccctc ccctcctcct accgcgtcag 14873
gaggggcgac atccgcggtt gacgcggcag cagatggtga ttacgaaccc ccgcggcgcc 14933
gggcccggca ctacctggac ttggaggagg gcgagggcct ggcgcggcta ggagcgccct 14993
ctcctgagcg gcacccaagg gtgcagctga agcgtgatac gcgtgaggcg tacgtgccgc 15053
ggcagaacct gtttcgcgac cgcgagggag aggagcccga ggagatgcgg gatcgaaagt 15113
tccacgcagg gcgcgagctg cggcatggcc tgaatcgcga gcggttgctg cgcgaggagg 15173
actttgagcc cgacgcgcga accgggatta gtcccgcgcg cgcacacgtg gcggccgccg 15233
acctggtaac cgcatacgag cagacggtga accaggagat taactttcaa aaaagcttta 15293
acaaccacgt gcgtacgctt gtggcgcgcg aggaggtggc tataggactg atgcatctgt 15353
gggactttgt aagcgcgctg gagcaaaacc caaatagcaa gccgctcatg gcgcagctgt 15413
tccttatagt gcagcacagc agggacaacg aggcattcag ggatgcgctg ctaaacatag 15473
tagagcccga gggccgctgg ctgctcgatt tgataaacat cctgcagagc atagtggtgc 15533
aggagcgcag cttgagcctg gctgacaagg tggccgccat caactattcc atgcttagcc 15593
tgggcaagtt ttacgcccgc aagatatacc atacccctta cgttcccata gacaaggagg 15653
taaagatcga ggggttctac atgcgcatgg cgctgaaggt gcttaccttg agcgacgacc 15713
tgggcgttta tcgcaacgag cgcatccaca aggccgtgag cgtgagccgg cggcgcgagc 15773
tcagcgaccg cgagctgatg cacagcctgc aaagggccct ggctggcacg ggcagcggcg 15833
atagagaggc cgagtcctac tttgacgcgg gcgctgacct gcgctgggcc ccaagccgac 15893
gcgccctgga ggcagctggg gccggacctg ggctggcggt ggcacccgcg cgcgctggca 15953
acgtcggcgg cgtggaggaa tatgacgagg acgatgagta cgagccagag gacggcgagt 16013

actaagcggt gatgtttctg atcagatgat gcaagacgca acggacccgg cggtgcgggc 16073

ggcgctgcag agccagccgt ccggccttaa ctccacggac gactggcgcc aggtcatgga 16133

ccgcatcatg tcgctgactg cgcgcaatcc tgacgcgttc cggcagcagc cgcaggccaa 16193

ccggctctcc gcaattctgg aagcggtggt cccggcgcgc gcaaacccca cgcacgagaa 16253

ggtgctggcg atcgtaaacg cgctggccga aaacagggcc atccggcccg acgaggccgg 16313

cctggtctac gacgcgctgc ttcagcgcgt ggctcgttac aacagcggca acgtgcagac 16373

caacctggac cggctggtgg gggatgtgcg cgaggccgtg gcgcagcgtg agcgcgcgca 16433

gcagcagggc aacctgggct ccatggttgc actaaacgcc ttcctgagta cacagcccgc 16493

caacgtgccg cggggacagg aggactacac caactttgtg agcgcactgc ggctaatggt 16553

gactgagaca ccgcaaagtg aggtgtacca gtctgggcca gactattttt tccagaccag 16613

tagacaaggc ctgcagaccg taaacctgag ccaggctttc aaaaacttgc aggggctgtg 16673

gggggtgcgg gctcccacag gcgaccgcgc gaccgtgtct agcttgctga cgcccaactc 16733

gcgcctgttg ctgctgctaa tagcgccctt cacggacagt ggcagcgtgt cccgggacac 16793

atacctaggt cacttgctga cactgtaccg cgaggccata ggtcaggcgc atgtggacga 16853

gcatactttc caggagatta caagtgtcag ccgcgcgctg gggcaggagg acacgggcag 16913

cctggaggca accctaaact acctgctgac caaccggcgg cagaagatcc cctcgttgca 16973

cagtttaaac agcgaggagg agcgcatttt gcgctacgtg cagcagagcg tgagccttaa 17033

cctgatgcgc gacggggtaa cgcccagcgt ggcgctggac atgaccgcgc gcaacatgga 17093

accgggcatg tatgcctcaa accggccgtt tatcaaccgc ctaatggact acttgcatcg 17153

cgcggccgcc gtgaaccccg agtatttcac caatgccatc ttgaacccgc actggctacc 17213

gccccctggt ttctacaccg ggggattcga ggtgcccgag ggtaacgatg gattcctctg 17273

ggacgacata gacgacagcg tgttttcccc gcaaccgcag accctgctag agttgcaaca 17333

gcgcgagcag gcagaggcgg cgctgcgaaa ggaaagcttc cgcaggccaa gcagcttgtc 17393

cgatctaggc gctgcggccc cgcggtcaga tgctagtagc ccatttccaa gcttgatagg 17453

gtctcttacc agcactcgca ccacccgccc gcgcctgctg ggcgaggagg agtacctaaa 17513

caactcgctg ctgcagccgc agcgcgaaaa aaacctgcct ccggcatttc ccaacaacgg 17573

gatagagagc ctagtggaca agatgagtag atggaagacg tacgcgcagg agcacaggga 17633

cgtgccaggc ccgcgcccgc ccacccgtcg tcaaaggcac gaccgtcagc ggggtctggt 17693

gtgggaggac gatgactcgg cagacgacag cagcgtcctg gatttgggag ggagtggcaa 17753

cccgtttgcg caccttcgcc ccaggctggg gagaatgttt taaaaaaaaa aaaagcatga 17813

tgcaaaataa aaaactcacc aaggccatgg caccgagcgt tggttttctt gtattcccct 17873

tagtatgcgg cgcgcggcga tgtatgagga aggtcctcct ccctcctacg agagtgtggt 17933

gagcgcggcg ccagtggcgg cggcgctggg ttctcccttc gatgctcccc tggacccgcc 17993

gtttgtgcct ccgcggtacc tgcggcctac cgggggagaa aacagcatcc gttactctga 18053

gttggcaccc ctattcgaca ccacccgtgt gtacctggtg gacaacaagt caacggatgt 18113

ggcatccctg aactaccaga acgaccacag caactttctg accacggtca ttcaaaacaa 18173

tgactacagc ccgggggagg caagcacaca gaccatcaat cttgacgacc ggtcgcactg 18233

gggcggcgac ctgaaaacca tcctgcatac caacatgcca aatgtgaacg agttcatgtt 18293

taccaataag tttaaggcgc gggtgatggt gtcgcgcttg cctactaagg acaatcaggt 18353

ggagctgaaa tacgagtggg tggagttcac gctgcccgag ggcaactact ccgagaccat 18413

gaccatagac cttatgaaca acgcgatcgt ggagcactac ttgaaagtgg gcagacagaa 18473

cggggttctg gaaagcgaca tcggggtaaa gtttgacacc cgcaacttca gactggggtt 18533

tgaccccgtc actggtcttg tcatgcctgg ggtatataca aacgaagcct tccatccaga 18593

catcattttg ctgccaggat gcggggtgga cttcacccac agccgcctga gcaacttgtt 18653

gggcatccgc aagcggcaac ccttccagga gggctttagg atcacctacg atgatctgga 18713

gggtggtaac attcccgcac tgttggatgt ggacgcctac caggcgagct tgaaagatga 18773

caccgaacag ggcgggggtg gcgcaggcgg cagcaacagc agtggcagcg gcgcggaaga 18833

gaactccaac gcggcagccg cggcaatgca gccggtggag gacatgaacg atcatgccat 18893

tcgcggcgac acctttgcca cacgggctga ggagaagcgc gctgaggccg aagcagcggc 18953

cgaagctgcc gcccccgctg cgcaacccga ggtcgagaag cctcagaaga aaccggtgat 19013

caaacccctg acagaggaca gcaagaaacg cagttacaac ctaataagca atgacagcac 19073

cttcacccag taccgcagct ggtaccttgc atacaactac ggcgaccctc agaccggaat 19133

ccgctcatgg accctgcttt gcactcctga cgtaacctgc ggctcggagc aggtctactg 19193

gtcgttgcca gacatgatgc aagaccccgt gaccttccgc tccacgcgcc agatcagcaa 19253

ctttccggtg gtgggcgccg agctgttgcc cgtgcactcc aagagcttct acaacgacca 19313

ggccgtctac tcccaactca tccgccagtt tacctctctg acccacgtgt tcaatcgctt 19373

tcccgagaac cagattttgg cgcgcccgcc agcccccacc atcaccaccg tcagtgaaaa 19433

cgttcctgct ctcacagatc acgggacgct accgctgcgc aacagcatcg gaggagtcca 19493

gcgagtgacc attactgacg ccagacgccg cacctgcccc tacgtttaca aggccctggg 19553

catagtctcg ccgcgcgtcc tatcgagccg cactttttga gcaagcatgt ccatccttat 19613

atcgcccagc aataacacag gctggggcct gcgcttccca agcaagatgt ttggcggggc 19673

caagaagcgc tccgaccaac acccagtgcg cgtgcgcggg cactaccgcg cgccctgggg 19733

cgcgcacaaa cgcggccgca ctgggcgcac caccgtcgat gacgccatcg acgcggtggt 19793

ggaggaggcg cgcaactaca cgcccacgcc gccaccagtg tccacagtgg acgcggccat 19853

tcagaccgtg gtgcgcggag cccggcgcta tgctaaaatg aagagacggc ggaggcgcgt 19913

agcacgtcgc caccgccgcc gacccggcac tgccgcccaa cgcgcggcgg cggccctgct 19973

taaccgcgca cgtcgcaccg gccgacgggc ggccatgcgg gccgctcgaa ggctggccgc 20033

gggtattgtc actgtgcccc ccaggtccag gcgacgagcg gccgccgcag cagccgcggc 20093

cattagtgct atgactcagg gtcgcagggg caacgtgtat tgggtgcgcg actcggttag 20153

cggcctgcgc gtgcccgtgc gcacccgccc cccgcgcaac tagattgcaa gaaaaaacta 20213

cttagactcg tactgttgta tgtatccagc ggcggcggcg cgcaacgaag ctatgtccaa 20273

gcgcaaaatc aaagaagaga tgctccaggt catcgcgccg gagatctatg gccccccgaa 20333

gaaggaagag caggattaca agccccgaaa gctaaagcgg gtcaaaaaga aaaagaaaga 20393

tgatgatgat gaacttgacg acgaggtgga actgctgcac gctaccgcgc ccaggcgacg 20453

ggtacagtgg aaaggtcgac gcgtaaaacg tgttttgcga cccggcacca ccgtagtctt 20513

tacgcccggt gagcgctcca cccgcaccta caagcgcgtg tatgatgagg tgtacggcga 20573

cgaggacctg cttgagcagg ccaacgagcg cctcggggag tttgcctacg gaaagcggca 20633

taaggacatg ctggcgttgc cgctggacga gggcaaccca acacctagcc taaagcccgt 20693

aacactgcag caggtgctgc ccgcgcttgc accgtccgaa gaaaagcgcg gcctaaagcg 20753

cgagtctggt gacttggcac ccaccgtgca gctgatggta cccaagcgcc agcgactgga 20813

agatgtcttg gaaaaaatga ccgtggaacc tgggctggag cccgaggtcc gcgtgcggcc 20873

aatcaagcag gtggcgccgg gactgggcgt gcagaccgtg gacgttcaga tacccactac 20933

cagtagcacc agtattgcca ccgccacaga gggcatggag acacaaacgt ccccggttgc 20993

ctcagcggtg gcggatgccg cggtgcaggc ggtcgctgcg gccgcgtcca agacctctac 21053

ggaggtgcaa acggacccgt ggatgtttcg cgtttcagcc ccccggcgcc cgcgccgttc 21113

gaggaagtac ggcgccgcca gcgcgctact gcccgaatat gccctacatc cttccattgc 21173

gcctaccccc ggctatcgtg gctacaccta ccgccccaga agacgagcaa ctacccgacg 21233

ccgaaccacc actggaaccc gccgccgccg tcgccgtcgc cagcccgtgc tggccccgat 21293

ttccgtgcgc agggtggctc gcgaaggagg caggaccctg gtgctgccaa cagcgcgcta 21353

ccaccccagc atcgtttaaa agccggtctt tgtggttctt gcagatatgg ccctcacctg 21413

ccgcctccgt ttcccggtgc cgggattccg aggaagaatg caccgtagga ggggcatggc 21473

cggccacggc ctgacgggcg gcatgcgtcg tgcgcaccac cggcggcggc gcgcgtcgca 21533

ccgtcgcatg cgcggcggta tcctgcccct ccttattcca ctgatcgccg cggcgattgg 21593

cgccgtgccc ggaattgcat ccgtggcctt gcaggcgcag agacactgat taaaaacaag 21653

ttgcatgtgg aaaaatcaaa ataaaaagtc tggactctca cgctcgcttg gtcctgtaac 21713

tattttgtag aatggaagac atcaactttg cgtctctggc cccgcgacac ggctcgcgcc 21773

cgttcatggg aaactggcaa gatatcggca ccagcaatat gagcggtggc gccttcagct 21833

ggggctcgct gtggagcggc attaaaaatt tcggttccac cgttaagaac tatggcagca 21893

aggcctggaa cagcagcaca ggccagatgc tgagggataa gttgaaagag caaaatttcc 21953

aacaaaaggt ggtagatggc ctggcctctg gcattagcgg ggtggtggac ctggccaacc 22013

aggcagtgca aaataagatt aacagtaagc ttgatccccg ccctcccgta gaggagcctc 22073

caccggccgt ggagacagtg tctccagagg ggcgtggcga aaagcgtccg cgccccgaca 22133

gggaagaaac tctggtgacg caaatagacg agcctccctc gtacgaggag gcactaaagc 22193

aaggcctgcc caccacccgt cccatcgcgc ccatggctac cggagtgctg ggccagcaca 22253

cacccgtaac gctggacctg cctccccccg ccgacaccca gcagaaacct gtgctgccag 22313

gcccgaccgc cgttgttgta acccgtccta gccgcgcgtc cctgcgccgc gccgccagcg 22373

gtccgcgatc gttgcggccc gtagccagtg gcaactggca aagcacactg aacagcatcg 22433

tgggtctggg ggtgcaatcc ctgaagcgcc gacgatgctt ctgatagcta acgtgtcgta 22493

tgtgtgtcat gtatgcgtcc atgtcgccgc cagaggagct gctgagccgc cgcgcgcccg 22553

ctttccaaga tggctacccc ttcgatgatg ccgcagtggt cttacatgca catctcgggc 22613

caggacgcct cggagtacct gagccccggg ctggtgcagt ttgcccgcgc caccgagacg 22673

tacttcagcc tgaataacaa gtttagaaac cccacggtgg cgcctacgca cgacgtgacc 22733

acagaccggt cccagcgttt gacgctgcgg ttcatccctg tggaccgtga ggatactgcg 22793

tactcgtaca aggcgcggtt caccctagct gtgggtgata accgtgtgct ggacatggct 22853

tccacgtact ttgacatccg cggcgtgctg gacagggccc ctacttttaa gccctactct 22913

ggcactgcct acaacgccct ggctcccaag ggtgccccaa atccttgcga atgggatgaa 22973

gctgctactg ctcttgaaat aaacctagaa gaagaggacg atgacaacga agacgaagta 23033

gacgagcaag ctgagcagca aaaaactcac gtatttgggc aggcgcctta ttctggtata 23093

aatattacaa aggagggtat tcaaataggt gtcgaaggtc aaacacctaa atatgccgat 23153

aaaacatttc aacctgaacc tcaaatagga gaatctcagt ggtacgaaac agaaattaat 23213

catgcagctg ggagagtcct aaaaaagact accccaatga aaccatgtta cggttcatat 23273

gcaaaaccca caaatgaaaa tggagggcaa ggcattcttg taaagcaaca aaatggaaag 23333

ctagaaagtc aagtggaaat gcaatttttc tcaactactg aggcagccgc aggcaatggt 23393

gataacttga ctcctaaagt ggtattgtac agtgaagatg tagatataga aaccccagac 23453

actcatattt cttacatgcc cactattaag gaaggtaact cacgagaact aatgggccaa 23513

caatctatgc ccaacaggcc taattacatt gcttttaggg acaattttat tggtctaatg 23573

tattacaaca gcacgggtaa tatgggtgtt ctggcgggcc aagcatcgca gttgaatgct 23633

gttgtagatt tgcaagacag aaacacagag ctttcatacc agcttttgct tgattccatt 23693

ggtgatagaa ccaggtactt ttctatgtgg aatcaggctg ttgacagcta tgatccagat 23753

gttagaatta ttgaaaatca tggaactgaa gatgaacttc caaattactg ctttccactg 23813

ggaggtgtga ttaatacaga gactcttacc aaggtaaaac ctaaaacagg tcaggaaaat 23873

ggatgggaaa aagatgctac agaattttca gataaaaatg aaataagagt tggaaataat 23933

tttgccatgg aaatcaatct aaatgccaac ctgtggagaa atttcctgta ctccaacata 23993

gcgctgtatt tgcccgacaa gctaaagtac agtccttcca acgtaaaaat ttctgataac 24053

ccaaacacct acgactacat gaacaagcga gtggtggctc ccgggctagt ggactgctac 24113

attaaccttg gagcacgctg gtcccttgac tatatggaca acgtcaaccc atttaaccac 24173

caccgcaatg ctggcctgcg ctaccgctca atgttgctgg gcaatggtcg ctatgtgccc 24233

ttccacatcc aggtgcctca gaagttcttt gccattaaaa acctccttct cctgccgggc 24293

tcatacacct acgagtggaa cttcaggaag gatgttaaca tggttctgca gagctcccta 24353

ggaaatgacc taagggttga cggagccagc attaagtttg atagcatttg cctttacgcc 24413

accttcttcc ccatggccca caacaccgcc tccacgcttg aggccatgct tagaaacgac 24473

accaacgacc agtcctttaa cgactatctc tccgccgcca acatgctcta ccctataccc 24533

gccaacgcta ccaacgtgcc catatccatc ccctcccgca actgggcggc tttccgcggc 24593

tgggccttca cgcgccttaa gactaaggaa accccatcac tgggctcggg ctacgaccct 24653

tattacacct actctggctc tataccctac ctagatggaa ccttttacct caaccacacc 24713

tttaagaagg tggccattac ctttgactct tctgtcagct ggcctggcaa tgaccgcctg 24773

cttaccccca acgagtttga aattaagcgc tcagttgacg gggagggtta caacgttgcc 24833

cagtgtaaca tgaccaaaga ctggttcctg gtacaaatgc tagctaacta taacattggc 24893

taccagggct tctatatccc agagagctac aaggaccgca tgtactcctt ctttagaaac 24953

ttccagccca tgagccgtca ggtggtggat gatactaaat acaaggacta ccaacaggtg 25013

ggcatcctac accaacacaa caactctgga tttgttggct accttgcccc caccatgcgc 25073

gaaggacagg cctaccctgc taacttcccc tatccgctta taggcaagac cgcagttgac 25133

agcattaccc agaaaaagtt tctttgcgat cgcacccttt ggcgcatccc attctccagt 25193

aactttatgt ccatgggcgc actcacagac ctgggccaaa accttctcta cgccaactcc 25253

gcccacgcgc tagacatgac ttttgaggtg gatcccatgg acgagcccac ccttctttat 25313

gttttgtttg aagtctttga cgtggtccgt gtgcaccagc cgcaccgcgg cgtcatcgaa 25373

accgtgtacc tgcgcacgcc cttctcggcc ggcaacgcca caacataaag aagcaagcaa 25433

catcaacaac agctgccgcc atgggctcca gtgagcagga actgaaagcc attgtcaaag 25493

atcttggttg tgggccatat tttttgggca cctatgacaa gcgctttcca ggctttgttt 25553

ctccacacaa gctcgcctgc gccatagtca atacggccgg tcgcgagact gggggcgtac 25613

actggatggc ctttgcctgg aacccgcact caaaaacatg ctacctcttt gagccctttg 25673

gcttttctga ccagcgactc aagcaggttt accagtttga gtacgagtca ctcctgcgcc 25733

gtagcgccat tgcttcttcc cccgaccgct gtataacgct ggaaaagtcc acccaaagcg 25793

tacaggggcc caactcggcc gcctgtggac tattctgctg catgtttctc cacgcctttg 25853

ccaactggcc ccaaactccc atggatcaca accccaccat gaaccttatt accggggtac 25913

ccaactccat gctcaacagt ccccaggtac agcccaccct gcgtcgcaac caggaacagc 25973

tctacagctt cctggagcgc cactcgccct acttccgcag ccacagtgcg cagattagga 26033

gcgccacttc tttttgtcac ttgaaaaaca tgtaaaaata atgtactaga gacactttca 26093

ataaaggcaa atgcttttat ttgtacactc tcgggtgatt atttaccccc acccttgccg 26153

tctgcgccgt ttaaaaatca aaggggttct gccgcgcatc gctatgcgcc actggcaggg 26213

acacgttgcg atactggtgt ttagtgctcc acttaaactc aggcacaacc atccgcggca 26273

gctcggtgaa gttttcactc cacaggctgc gcaccatcac caacgcgttt agcaggtcgg 26333

gcgccgatat cttgaagtcg cagttggggc ctccgccctg cgcgcgcgag ttgcgataca 26393

cagggttgca gcactggaac actatcagcg ccgggtggtg cacgctggcc agcacgctct 26453

tgtcggagat cagatccgcg tccaggtcct ccgcgttgct cagggcgaac ggagtcaact 26513

ttggtagctg ccttcccaaa aagggcgcgt gcccaggctt tgagttgcac tcgcaccgta 26573

gtggcatcaa aaggtgaccg tgcccggtct gggcgttagg atacagcgcc tgcataaaag 26633

ccttgatctg cttaaaagcc acctgagcct ttgcgccttc agagaagaac atgccgcaag 26693

acttgccgga aaactgattg gccggacagg ccgcgtcgtg cacgcagcac cttgcgtcgg 26753

tgttggagat ctgcaccaca tttcggcccc accggttctt cacgatcttg gccttgctag 26813

actgctcctt cagcgcgcgc tgcccgtttt cgctcgtcac atccatttca atcacgtgct 26873

ccttatttat cataatgctt ccgtgtagac acttaagctc gccttcgatc tcagcgcagc 26933

ggtgcagcca caacgcgcag cccgtgggct cgtgatgctt gtaggtcacc tctgcaaacg 26993

actgcaggta cgcctgcagg aatcgcccca tcatcgtcac aaaggtcttg ttgctggtga 27053

aggtcagctg caacccgcgg tgctcctcgt tcagccaggt cttgcatacg gccgccagag 27113

cttccacttg gtcaggcagt agtttgaagt tcgcctttag atcgttatcc acgtggtact 27173

tgtccatcag cgcgcgcgca gcctccatgc ccttctccca cgcagacacg atcggcacac 27233

tcagcgggtt catcaccgta atttcacttt ccgcttcgct gggctcttcc tcttcctctt 27293

gcgtccgcat accacgcgcc actgggtcgt cttcattcag ccgccgcact gtgcgcttac 27353

ctcctttgcc atgcttgatt agcaccggtg ggttgctgaa acccaccatt tgtagcgcca 27413

catcttctct ttcttcctcg ctgtccacga ttacctctgg tgatggcggg cgctcgggct 27473

tgggagaagg gcgcttcttt ttcttcttgg gcgcaatggc caaatccgcc gccgaggtcg 27533

atggccgcgg gctgggtgtg cgcggcacca gcgcgtcttg tgatgagtct tcctcgtcct 27593

cggactcgat acgccgcctc atccgctttt ttgggggcgc ccggggaggc ggcggcgacg 27653

gggacgggga cgacacgtcc tccatggttg ggggacgtcg cgccgcaccg cgtccgcgct 27713

cgggggtggt ttcgcgctgc tcctcttccc gactggccat ttccttctcc tataggcaga 27773

aaaagatcat ggagtcagtc gagaagaagg acagcctaac cgccccctct gagttcgcca 27833

ccaccgcctc caccgatgcc gccaacgcgc ctaccacctt ccccgtcgag gcacccccgc 27893

ttgaggagga ggaagtgatt atcgagcagg acccaggttt tgtaagcgaa gacgacgagg 27953

accgctcagt accaacagag gataaaaagc aagaccagga caacgcagag gcaaacgagg 28013

aacaagtcgg gcggggggac gaaaggcatg gcgactacct agatgtggga gacgacgtgc 28073

tgttgaagca tctgcagcgc cagtgcgcca ttatctgcga cgcgttgcaa gagcgcagcg 28133

atgtgcccct cgccatagcg gatgtcagcc ttgcctacga acgccaccta ttctcaccgc 28193

gcgtaccccc caaacgccaa gaaaacggca catgcgagcc caacccgcgc ctcaacttct 28253

accccgtatt tgccgtgcca gaggtgcttg ccacctatca catctttttc caaaactgca 28313

agatacccct atcctgccgt gccaaccgca gccgagcgga caagcagctg gccttgcggc 28373

agggcgctgt catacctgat atcgcctcgc tcaacgaagt gccaaaaatc tttgagggtc 28433

ttggacgcga cgagaagcgc gcggcaaacg ctctgcaaca ggaaaacagc gaaaatgaaa 28493

gtcactctgg agtgttggtg gaactcgagg gtgacaacgc gcgcctagcc gtactaaaac 28553

gcagcatcga ggtcacccac tttgcctacc cggcacttaa cctacccccc aaggtcatga 28613

gcacagtcat gagtgagctg atcgtgcgcc gtgcgcagcc cctggagagg gatgcaaatt 28673

tgcaagaaca aacagaggag ggcctacccg cagttggcga cgagcagcta gcgcgctggc 28733

ttcaaacgcg cgagcctgcc gacttggagg agcgacgcaa actaatgatg gccgcagtgc 28793

tcgttaccgt ggagcttgag tgcatgcagc ggttctttgc tgacccggag atgcagcgca 28853

agctagagga aacattgcac tacacctttc gacagggcta cgtacgccag gcctgcaaga 28913

tctccaacgt ggagctctgc aacctggtct cctaccttgg aattttgcac gaaaaccgcc 28973

ttgggcaaaa cgtgcttcat tccacgctca agggcgaggc gcgccgcgac tacgtccgcg 29033

actgcgttta cttatttcta tgctacacct ggcagacggc catgggcgtt tggcagcagt 29093

gcttggagga gtgcaacctc aaggagctgc agaaactgct aaagcaaaac ttgaaggacc 29153

tatggacggc cttcaacgag cgctccgtgg ccgcgcacct ggcggacatc attttccccg 29213

aacgcctgct taaaaccctg caacagggtc tgccagactt caccagtcaa agcatgttgc 29273

agaactttag gaactttatc ctagagcgct caggaatctt gcccgccacc tgctgtgcac 29333

ttcctagcga ctttgtgccc attaagtacc gcgaatgccc tccgccgctt tggggccact 29393

gctaccttct gcagctagcc aactaccttg cctaccactc tgacataatg gaagacgtga 29453

gcggtgacgg tctactggag tgtcactgtc gctgcaacct atgcaccccg caccgctccc 29513

tggtttgcaa ttcgcagctg cttaacgaaa gtcaaattat cggtaccttt gagctgcagg 29573

gtccctcgcc tgacgaaaag tccgcggctc cggggttgaa actcactccg gggctgtgga 29633

cgtcggctta ccttcgcaaa tttgtacctg aggactacca cgcccacgag attaggttct 29693

acgaagacca atcccgcccg cctaatgcgg agcttaccgc ctgcgtcatt acccagggcc 29753

acattcttgg ccaattgcaa gccatcaaca aagcccgcca agagtttctg ctacgaaagg 29813

gacggggggt ttacttggac ccccagtccg gcgaggagct caacccaatc cccccgccgc 29873

cgcagcccta tcagcagcag ccgcgggccc ttgcttccca ggatggcacc caaaaagaag 29933

ctgcagctgc cgccgccacc cacggacgag gaggaatact gggacagtca ggcagaggag 29993

gttttggacg aggaggagga ggacatgatg gaagactggg agagcctaga cgaggaagct 30053

tccgaggtcg aagaggtgtc agacgaaaca ccgtcaccct cggtcgcatt cccctcgccg 30113

gcgccccaga aatcggcaac cggttccagc atggctacaa cctccgctcc tcaggcgccg 30173

ccggcactgc ccgttcgccg acccaaccgt agatgggaca ccactggaac cagggccggt 30233

aagtccaagc agccgccgcc gttagcccaa gagcaacaac agcgccaagg ctaccgctca 30293

tggcgcgggc acaagaacgc catagttgct tgcttgcaag actgtggggg caacatctcc 30353

ttcgcccgcc gctttcttct ctaccatcac ggcgtggcct tcccccgtaa catcctgcat 30413

tactaccgtc atctctacag cccatactgc accggcggca gcggcagcgg cagcaacagc 30473

agcggccaca cagaagcaaa ggcgaccgga tagcaagact ctgacaaagc ccaagaaatc 30533

cacagcggcg gcagcagcag gaggaggagc gctgcgtctg gcgcccaacg aacccgtatc 30593

gacccgcgag cttagaaaca ggatttttcc cactctgtat gctatatttc aacagagcag 30653

gggccaagaa caagagctga aaataaaaaa caggtctctg cgatccctca cccgcagctg 30713

cctgtatcac aaaagcgaag atcagcttcg gcgcacgctg gaagacgcgg aggctctctt 30773

cagtaaatac tgcgcgctga ctcttaagga ctagtttcgc gccctttctc aaatttaagc 30833

gcgaaaacta cgtcatctcc agcggccaca cccggcgcca gcacctgtcg tcagcgccat 30893

tatgagcaag gaaattccca cgccctacat gtggagttac cagccacaaa tgggacttgc 30953

ggctggagct gcccaagact actcaacccg aataaactac atgagcgcgg gaccccacat 31013

gatatcccgg gtcaacggaa tacgcgccca ccgaaaccga attctcttgg aacaggcggc 31073

tattaccacc acacctcgta ataaccttaa tccccgtagt tggcccgctg ccctggtgta 31133

ccaggaaagt cccgctccca ccactgtggt acttcccaga gacgcccagg ccgaagttca 31193

gatgactaac tcaggggcgc agcttgcggg cggctttcgt cacagggtgc ggtcgcccgg 31253

gcagggtata actcacctga caatcagagg gcgaggtatt cagctcaacg acgagtcggt 31313

gagctcctcg cttggtctcc gtccggacgg gacatttcag atcggcggcg ccggccgctc 31373

ttcattcacg cctcgtcagg caatcctaac tctgcagacc tcgtcctctg agccgcgctc 31433

tggaggcatt ggaactctgc aatttattga ggagtttgtg ccatcggtct actttaaccc 31493

cttctcggga cctcccggcc actatccgga tcaatttatt cctaactttg acgcggtaaa 31553

ggactcggcg gacggctacg actgaatgtt aagtggagag gcagagcaac tgcgcctgaa 31613

acacctggtc cactgtcgcc gccacaagtg ctttgcccgc gactccggtg agttttgcta 31673

ctttgaattg cccgaggatc atatcgaggg cccggcgcac ggcgtccggc ttaccgccca 31733

gggagagctt gcccgtagcc tgattcggga gtttacccag cgccccctgc tagttgagcg 31793

ggacagggga ccctgtgttc tcactgtgat ttgcaactgt cctaaccctg gattacatca 31853

agatcctcta gttataacta gagtacccgg ggatcttatt ccctttaact aataaaaaaa 31913

aataataaag catcacttac ttaaaatcag ttagcaaatt tctgtccagt ttattcagca 31973

gcacctcctt gccctcctcc cagctctggt attgcagctt cctcctggct gcaaactttc 32033

tccacaatct aaatggaatg tcagtttcct cctgttcctg tccatccgca cccactatct 32093

tcatgttgtt gcagatgaag cgcgcaagac cgtctgaaga taccttcaac cccgtgtatc 32153

catatgacac ggaaaccggt cctccaactg tgccttttct tactcctccc tttgtatccc 32213

ccaatgggtt tcaagagagt ccccctgggg tactctcttt gcgcctatcc gaacctctag 32273

ttacctccaa tggcatgctt gcgctcaaaa tgggcaacgg cctctctctg gacgaggccg 32333

gcaaccttac ctcccaaaat gtaaccactg tgagcccacc tctcaaaaaa accaagtcaa 32393

acataaacct ggaaatatct gcacccctca cagttacctc agaagcccta actgtggctg 32453

ccgccgcacc tctaatggtc gcgggcaaca cactcaccat gcaatcacag gccccgctaa 32513

ccgtgcacga ctccaaactt agcattgcca cccaaggacc cctcacagtg tcagaaggaa 32573

agctagccct gcaaacatca ggccccctca ccaccaccga tagcagtacc cttactatca 32633

ctgcctcacc ccctctaact actgccactg gtagcttggg cattgacttg aaagagccca 32693

tttatacaca aaatggaaaa ctaggactaa agtacggggc tcctttgcat gtaacagacg 32753

acctaaacac tttgaccgta gcaactggtc caggtgtgac tattaataat acttccttgc 32813

aaactaaagt tactggagcc ttgggttttg attcacaagg caatatgcaa cttaatgtag 32873

caggaggact aaggattgat tctcaaaaca gacgccttat acttgatgtt agttatccgt 32933

ttgatgctca aaaccaacta aatctaagac taggacaggg ccctcttttt ataaactcag 32993

cccacaactt ggatattaac tacaacaaag gcctttactt gtttacagct tcaaacaatt 33053

ccaaaaagct tgaggttaac ctaagcactg ccaaggggtt gatgtttgac gctacagcca 33113

tagccattaa tgcaggagat gggcttgaat ttggttcacc taatgcacca aacacaaatc 33173

ccctcaaaac aaaaattggc catggcctag aatttgattc aaacaaggct atggttccta 33233

aactaggaac tggccttagt tttgacagca caggtgccat tacagtagga aacaaaaata 33293

atgataagct aactttgtgg accacaccag ctccatctcc taactgtaga ctaaatgcag 33353

agaaagatgc taaactcact ttggtcttaa caaaatgtgg cagtcaaata cttgctacag 33413

tttcagtttt ggctgttaaa ggcagtttgg ctccaatatc tggaacagtt caaagtgctc 33473

atcttattat aagatttgac gaaaatggag tgctactaaa caattccttc ctggacccag 33533

aatattggaa ctttagaaat ggagatctta ctgaaggcac agcctataca aacgctgttg 33593

gatttatgcc taacctatca gcttatccaa aatctcacgg taaaactgcc aaaagtaaca 33653

ttgtcagtca agtttactta aacggagaca aaactaaacc tgtaacacta accattacac 33713

taaacggtac acaggaaaca ggagacacaa ctccaagtgc atactctatg tcattttcat 33773

gggactggtc tggccacaac tacattaatg aaatatttgc cacatcctct tacacttttt 33833

catacattgc ccaagaataa agaatcgttt gtgttatgtt tcaacgtgtt tatttttcaa 33893

ttgcagaaaa tttcaagtca tttttcattc agtagtatag ccccaccacc acatagctta 33953

tacagatcac cgtaccttaa tcaaactcac agaaccctag tattcaacct gccacctccc 34013

tcccaacaca cagagtacac agtcctttct ccccggctgg ccttaaaaag catcatatca 34073

tgggtaacag acatattctt aggtgttata ttccacacgg tttcctgtcg agccaaacgc 34133

tcatcagtga tattaataaa ctccccgggc agctcactta agttcatgtc gctgtccagc 34193

tgctgagcca caggctgctg tccaacttgc ggttgcttaa cgggcggcga aggagaagtc 34253

cacgcctaca tgggggtaga gtcataatcg tgcatcagga tagggcggtg gtgctgcagc 34313

agcgcgcgaa taaactgctg ccgccgccgc tccgtcctgc aggaatacaa catggcagtg 34373

gtctcctcag cgatgattcg caccgcccgc agcataaggc gccttgtcct ccgggcacag 34433

cagcgcaccc tgatctcact taaatcagca cagtaactgc agcacagcac cacaatattg 34493

ttcaaaatcc cacagtgcaa ggcgctgtat ccaaagctca tggcggggac cacagaaccc 34553

acgtggccat cataccacaa gcgcaggtag attaagtggc gacccctcat aaacacgctg 34613

gacataaaca ttacctcttt tggcatgttg taattcacca cctcccggta ccatataaac 34673

ctctgattaa acatggcgcc atccaccacc atcctaaacc agctggccaa aacctgcccg 34733

ccggctatac actgcaggga accgggactg gaacaatgac agtggagagc ccaggactcg 34793

taaccatgga tcatcatgct cgtcatgata tcaatgttgg cacaacacag gcacacgtgc 34853

atacacttcc tcaagattac aagctcctcc cgcgttagaa ccatatccca gggaacaacc 34913

cattcctgaa tcagcgtaaa tcccacactg cagggaagac ctcgcacgta actcacgttg 34973

tgcattgtca aagtgttaca ttcgggcagc agcggatgat cctccagtat ggtagcgcgg 35033

gtttctgtct caaaaggagg tagacgatcc ctactgtacg gagtgcgccg agacaaccga 35093

gatcgtgttg gtcgtagtgt catgccaaat ggaacgccgg acgtagtcat atttcctgaa 35153

gcaaaaccag gtgcgggcgt gacaaacaga tctgcgtctc cggtcctgcc gcttagcgtg 35213

ttccgtgtga tagttcaagt acaaccacac tcttaagttg gtcaaaagaa tgctggcttc 35273

agttgtaatc aaaactccat cgcatctaat cgttctgagg aaatcatcca cggtagcata 35333

tgcaaatccc aaccaagcaa tgcaactgga ttgtgtttca agcaggagag gagagggaag 35393

agacggaaga accatgttaa tttttttatt ccaaaagatt atccaaaacc tcaaaatgaa 35453

gatctattaa gtgaacgcgc tcccctccgg tggcgtggtc aaactctaca gccaaagaac 35513

agataatggc atttgtaaga tgttgcacaa tggcttccaa aaggcaaacg gccctcacgt 35573

ccaagtggac gtaaaggcta aacccttcag ggtgaatctc ctctataaac attccagcac 35633

cttcaaccat gcccaaataa ttctcatctc gccaccttct caatatatct ctaagcaaat 35693

cccgaatatt aagtccggcc attgtaaaaa tctgctccag agcgccctcc accttacagc 35753

ctcaagcagc gaatcatgat tgcaaaaatt caggttcctc acagacctgt ataagattca 35813

aaagcggaac attaacaaaa ataccgcgat cccgtaggtc ccttcgcagg gccagctgaa 35873

cataatcgtg caggtctgca cggaccagcg cggccacttc cccgccagga accatgacaa 35933

aagaacccac actgattatg acacgcatac tcggagctat gctaaccagc gtagccccga 35993

tgtaagcttg ttgcatgggc ggcgatataa aatgcaaggt gctgctcaaa aaatcaggca 36053

aagcctcgcg caaaaaagaa agcacatcgt agtcatgctc atgcagataa aggcaggtaa 36113

gctccggaac caccacagaa aaagacacca tttttctctc aaacatgtct gcgggtttct 36173

gcataaacac aaaataaaat aacaaaaaaa catttaaaca ttagaagcct gtcttacaac 36233

aggaaaaaca acccttataa gcataagacg gactacggcc atgccggcgt gaccgtaaaa 36293

aaactggtca ccgtgattaa aaagcaccac cgacagctcc tcggtcatgt ccggagtcat 36353

aatgtaagac tcggtaaaca catcaggttg attcacatcg gtcagtgcta aaaagcgacc 36413

gaaatagccc gggggaatac atacccgcag gcgtagagac aacattacag cccccatagg 36473

aggtataaca aaattaatag gagagaaaaa cacataaaca cctgaaaaac cctcctgcct 36533

aggcaaaata gcaccctccc gctccagaac aacatacagc gcttccacag cggcagccat 36593

aacagtcagc cttaccagta aaaaagaaaa cctattaaaa aaacaccact cgacacggca 36653

ccagctcaat cagtcacagt gtaaaaaagg gccaagtgca gagcgagtat atataggact 36713

aaaaaatgac gtaacggtta aagtccacaa aaaacaccca gaaaaccgca cgcgaaccta 36773

cgcccagaaa cgaaagccaa aaaacccaca acttcctcaa atcgtcactt ccgttttccc 36833

acgttacgtc acttcccatt ttaagaaaac tacaattccc aacacataca agttactccg 36893

ccctaaaacc tacgtcaccc gccccgttcc cacgccccgc gccacgtcac aaactccacc 36953

ccctcattat catattggct tcaatccaaa ataaggtata ttattgatga tgttacatcg 37013

ttaattaa 37021

<210> 5
<211> 235
<212> PRT
<213> Artificial Sequence

<220>
<223> [CDS]:4349..5053 from SEQ ID NO 4

<400> 5
Met Thr Val Leu Ala Pro Ala Trp Ser Pro Thr Thr Tyr Leu Leu Leu
1               5                   10                  15
Leu Leu Leu Leu Ser Ser Gly Leu Ser Gly Thr Gln Asp Cys Ser Phe
            20                  25                  30
Gln His Ser Pro Ile Ser Ser Asp Phe Ala Val Lys Ile Arg Glu Leu
        35                  40                  45
Ser Asp Tyr Leu Leu Gln Asp Tyr Pro Val Thr Val Ala Ser Asn Leu
    50                  55                  60
Gln Asp Glu Glu Leu Cys Gly Gly Leu Trp Arg Leu Val Leu Ala Gln
65                  70                  75                  80
Arg Trp Met Glu Arg Leu Lys Thr Val Ala Gly Ser Lys Met Gln Gly
                85                  90                  95
Leu Leu Glu Arg Val Asn Thr Glu Ile His Phe Val Thr Lys Cys Ala
            100                 105                 110
Phe Gln Pro Pro Pro Ser Cys Leu Arg Phe Val Gln Thr Asn Ile Ser
        115                 120                 125
Arg Leu Leu Gln Glu Thr Ser Glu Gln Leu Val Ala Leu Lys Pro Trp
    130                 135                 140
Ile Thr Arg Gln Asn Phe Ser Arg Cys Leu Glu Leu Gln Cys Gln Pro
145                 150                 155                 160
Asp Ser Ser Thr Leu Pro Pro Pro Trp Ser Pro Arg Pro Leu Glu Ala
                165                 170                 175
Thr Ala Pro Thr Ala Pro Gln Pro Pro Leu Leu Leu Leu Leu Leu Leu
            180                 185                 190
Pro Val Gly Leu Leu Leu Leu Ala Ala Ala Trp Cys Leu His Trp Gln
        195                 200                 205
Arg Thr Arg Arg Arg Thr Pro Arg Pro Gly Glu Gln Val Pro Pro Val
    210                 215                 220
Pro Ser Pro Gln Asp Leu Leu Leu Val Glu His
225                 230                 235

<210> 6
<211> 37077
<212> DNA
<213> Artificial Sequence

<220>
<223> Ad5 including E4 orf4-encoded protein

<220>
<221> LTR
<222> 1..103

<223> /note="L-ITR"

<220>
<221> promoter
<222> 321..963
<223> /note="CMVgal promoter"

<220>
<221> gene
<222> 1037..1956
<223> /note="E1A (delta aa 4-25)"

<220>
<221> misc_feature
<222> 1147..1167
<223> /note="RNAi target site"

<220>
<221> misc_feature
<222> 1196..1219
<223> /note="RNAi target site"

<220>
<221> misc_feature
<222> 1994..2074
<223> /note="heterologous target sites for RNAi inserted into the 3´UTR
      of E1A, equivalent to E1A-1196..1219, E1A-1147..1167, Prot
      IX-7409..7429"

<220>
<221> promoter
<222> 2084..2220
<223> /note="promoter for E1B"

<220>
<221> gene
<222> 2221..2751
<223> /note="E1B-19k"

<220>
<221> gene
<222> 2526..4016
<223> /note="E1B-55k"

<220>
<221> misc_signal
<222> 3421..4630
<223> /note="IRES"

<220>
<221> gene
<222> 4659..5363
<223> /note="FLT3L"

<220>
<221> misc_feature
<222> 5364..5426
<223> /note="2A"

<220>
<221> gene
<222> 5427..5702
<223> /note="MIP1A/CCL3"

<220>
<221> misc_feature
<222> 5703..5765
<223> /note="2A"

<220>
<221> gene
<222> 5766..6110
<223> /note="XCL1"

<220>
<221> promoter
<222> 6182..6538
<223> /note="prMinRGC"

<220>
<221> gene
<222> 6539..7144
<223> /note="GAL4-KRAB"

<220>
<221> gene
<222> 7388..7810
<223> /note="Protein IX"

<220>
<221> gene
<222> 8976..17898
<223> /note="Ad-Pol"

<220>
<221> gene
<222> 12362..17898
<223> /note="terminal protein TP"

<220>
<221> gene
<222> 17935..19650
<223> /note="Protein III"

<220>
<221> gene
<222> 22621..25479
<223> /note="hexon"

<220>
<221> gene
<222> 26222..27811
<223> /note="reverse complementary sequence of DNA-binding protein,
      dbd"

<220>
<221> gene
<222> 27840..30263
<223> /note="100k"

<220>
<221> misc_feature
<222> 31645
<223> /note="delta E3"

<220>

<221> gene
<222> 32164..33909
<223> /note="fiber"

<220>
<221> gene
<222> 34036..35199
<223> /note="reverse complementary sequence for E4 orf6/7"

<220>
<221> gene
<222> 35120..35464
<223> /note="reverse complementary sequence for hybrid E4orf4"

<220>
<221> misc_feature
<222> 35475..35826
<223> /note="inactive E4 orf3 locus"

<220>
<221> misc_feature
<222> 35803..35805
<223> /note="stop codon for E4orf3"

<220>
<221> gene
<222> 35838..36231
<223> /note="reverse complementary sequence for E4 orf2"

<220>
<221> gene
<222> 36263..36649
<223> /note="reverse complementary sequence for E4 orf1"

<220>
<221> LTR
<222> 36974..37077
<223> /note="R-ITR"

<400> 6
catcatcaat aatatacctt attttggatt gaagccaata tgataatgag ggggtggagt 60

ttgtgacgtg gcgcggggcg tgggaacggg gcgggtgacg tagtagtgtg gcggaagtgt 120

gatgttgcaa gtgtggcgga acacatgtaa gcgacggatg tggcaaaagt gacgtttttg 180

gtgtgcgccg gtgtacacag gaagtgacaa ttttcgcgcg gttttaggcg gatgttgtag 240

taaatttggg cgtaaccgag taagatttgg ccattttcgc gggaaaactg aataagagga 300

agtgaaatct gaataatttt cccgggtacc tataccctct agagtctccg ctcggaggac 360

agtactccgc tcggaggaca gtactccgct cggaggacag tactccgctc ggaggacagt 420

actccgctcg gaggacagta ctccgacctg caggcatgca aggtacctat accctctaga 480

gtctccgctc ggaggacagt actccgctcg gaggacagta ctccgctcgg aggacagtac 540

tccgctcgga ggacagtact ccgctcggag gacagtactc cgacctgcag gcatgcaagg 600

taccatggtg atgcggtttt ggcagtacat caatggcggt ggatagcggt ccgactcacg 660

gggatttcca agtctccacc ccattgacgt caatgggagt ttgttttggc accaaaatca 720

acgggacttt ccaaaatgtc gtaacaactc cgccccacgg acgcaaatgg gccgtaggcg 780

tgtacggtgg gcggtctata taagcccggc tggtttagtg aaccgtcaga tcctcgacta 840

gagtctcgct cggaggacag tactccgctc ggaggacagt actccgctcg gaggacagta 900

ctccgctcgg aggacagtac tccgctcgga ggacagtact ccgacctgca ggcatgcaag 960

gttcctcaag aggccactct tgagtgccag cgagtagagt tttctcctcc gagccgctcc 1020

gacaccggga ctgaaaatga gacatgaggt actggctgat aatcttccac ctcctagcca 1080

ttttgaacca cctacccttc acgaactgta tgatttagac gtgacggccc ccgaagatcc 1140

caacgaggag gcggtttcgc agatttttcc cgactctgta atgttggcgg tgcaggaagg 1200

gattgactta ctcacttttc cgccggcgcc cggttctccg gagccgcctc acctttcccg 1260

gcagcccgag cagccggagc agagagcctt gggtccggtt tctatgccaa accttgtacc 1320

ggaggtgatc gatcttacct gccacgaggc tggctttcca cccagtgacg acgaggatga 1380

agagggtgag gagtttgtgt tagattatgt ggagcacccc gggcacggtt gcaggtcttg 1440

tcattatcac cggaggaata cgggggaccc agatattatg tgttcgcttt gctatatgag 1500

gacctgtggc atgtttgtct acagtaagtg aaaattatgg gcagtgggtg atagagtggt 1560

gggtttggtg tggtaatttt ttttttaatt tttacagttt tgtggtttaa agaattttgt 1620

attgtgattt ttttaaaagg tcctgtgtct gaacctgagc ctgagcccga gccagaaccg 1680

gagcctgcaa gacctacccg ccgtcctaaa atggcgcctg ctatcctgag acgcccgaca 1740

tcacctgtgt ctagagaatg caatagtagt acggatagct gtgactccgg tccttctaac 1800

acacctcctg agatacaccc ggtggtcccg ctgtgcccca ttaaaccagt tgccgtgaga 1860

gttggtgggc gtcgccaggc tgtggaatgt atcgaggact tgcttaacga gcctgggcaa 1920

cctttggact tgagctgtaa acgccccagg ccataaggtg taaacctgtg attgcgtgtg 1980

tggttaacac acaggaggcg gtttcgcaga tttttacaca cagaagggat tgacttactc 2040

acttttacac acaggaagca ttgtgagctc atatgagacc caacgccttt gtttgctgaa 2100

tgagttgatg taagtttaat aaagggtgag ataatgttta acttgcatgg cgtgttaaat 2160

ggggcggggc ttaaagggta tataatgcgc cgtgggctaa tcttggttac atctgacctc 2220

atggaggctt gggagtgttt ggaagatttt tctgctgtgc gtaacttgct ggaacagagc 2280

tctaacagta cctcttggtt ttggaggttt ctgtggggct catcccaggc aaagttagtc 2340

tgcagaatta aggaggatta caagtgggaa tttgaagagc ttttgaaatc ctgtggtgag 2400

ctgtttgatt ctttgaatct gggtcaccag gcgcttttcc aagagaaggt catcaagact 2460

ttggattttt ccacaccggg gcgcgctgcg gctgctgttg cttttttgag ttttataaag 2520

gataaatgga gcgaagaaac ccatctgagc ggggggtacc tgctggattt tctggccatg 2580

catctgtgga gagcggttgt gagacacaag aatcgcctgc tactgttgtc ttccgtccgc 2640

ccggcgataa taccgacgga ggagcagcag cagcagcagg aggaagccag gcggcggcgg 2700

caggagcaga gcccatggaa cccgagagcc ggcctggacc ctcgggaatg aatgttgtac 2760

aggtggctga actgtatcca gaactgagac gcattttgac aattacagag gatgggcagg 2820

ggctaaaggg ggtaaagagg gagcgggggg cttgtgaggc tacagaggag gctaggaatc 2880

tagcttttag cttaatgacc agacaccgtc ctgagtgtat tacttttcaa cagatcaagg 2940

ataattgcgc taatgagctt gatctgctgg cgcagaagta ttccatagag cagctgacca 3000

cttactggct gcagccaggg gatgattttg aggaggctat tagggtatat gcaaaggtgg 3060

cacttaggcc agattgcaag tacaagatca gcaaacttgt aaatatcagg aattgttgct 3120

acatttctgg gaacggggcc gaggtggaga tagatacgga ggatagggtg gcctttagat 3180

gtagcatgat aaatatgtgg ccgggggtgc ttggcatgga cggggtggtt attatgaatg 3240

taaggtttac tggccccaat tttagcggta cggttttcct ggccaatacc aaccttatcc 3300

tacacggtgt aagcttctat gggtttaaca atacctgtgt ggaagcctgg accgatgtaa 3360

gggttcgggg ctgtgccttt tactgctgct ggaagggggt ggtgtgtcgc cccaaaagca 3420

gggcttcaat taagaaatgc ctctttgaaa ggtgtacctt gggtatcctg tctgagggta 3480

actccagggt gcgccacaat gtggcctccg actgtggttg cttcatgcta gtgaaaagcg 3540

tggctgtgat taagcataac atggtatgtg gcaactgcga ggacagggcc tctcagatgc 3600

tgacctgctc ggacggcaac tgtcacctgc tgaagaccat tcacgtagcc agccactctc 3660

gcaaggcctg gccagtgttt gagcataaca tactgacccg ctgttccttg catttgggta 3720

acaggagggg ggtgttccta ccttaccaat gcaatttgag tcacactaag atattgcttg 3780

agcccgagag catgtccaag gtgaacctga acggggtgtt tgacatgacc atgaagatct 3840

ggaaggtgct gaggtacgat gagacccgca ccaggtgcag accctgcgag tgtggcggta 3900

aacatattag gaaccagcct gtgatgctgg atgtgaccga ggagctgagg cccgatcact 3960

tggtgctggc ctgcacccgc gctgagtttg gctctagcga tgaagataca gattgaggta 4020

ctgaaatgtg tgggcgtggc gaattccgcc cctctccctc ccccccccct aacgttactg 4080

gccgaagccg cttggaataa ggccggtgtg cgtttgtcta tatgttattt tccaccatat 4140

tgccgtcttt tggcaatgtg agggcccgga aacctggccc tgtcttcttg acgagcattc 4200

ctaggggtct ttcccctctc gccaaaggaa tgcaaggtct gttgaatgtc gtgaaggaag 4260

cagttcctct ggaagcttct tgaagacaaa caacgtctgt agcgaccctt tgcaggcagc 4320

ggaacccccc acctggcgac aggtgcctct gcggccaaaa gccacgtgta taagatacac 4380

ctgcaaaggc ggcacaaccc cagtgccacg ttgtgagttg gatagttgtg gaaagagtca 4440

aatggctctc ctcaagcgta ttcaacaagg ggctgaagga tgcccagaag gtaccccatt 4500

gtatgggatc tgatctgggg cctcggtgca catgctttac atgtgtttag tcgaggttaa 4560

aaaaacgtct aggccccccg aaccacgggg acgtggtttt cctttgaaaa acacgatgat 4620

aagcttgatc acgcgtttcg aacgatttaa atttaaatat gacagtgctg gcgccagcct 4680

ggagcccaac aacctatctc ctcctgctgc tgctgctgag ctcgggactc agtgggaccc 4740

aggactgctc cttccaacac agccccatct cctccgactt cgctgtcaaa atccgtgagc 4800

tgtctgacta cctgcttcaa gattacccag tcaccgtggc ctccaacctg caggacgagg 4860

agctctgcgg gggcctctgg cggctggtcc tggcacagcg ctggatggag cggctcaaga 4920

ctgtcgctgg gtccaagatg caaggcttgc tggagcgcgt gaacacggag atacactttg 4980

tcaccaaatg tgcctttcag ccccccccca gctgtcttcg cttcgtccag accaacatct 5040

cccgcctcct gcaggagacc tccgagcagc tggtggcgct gaagccctgg atcactcgcc 5100

agaacttctc ccggtgcctg gagctgcagt gtcagcccga ctcctcaacc ctgccacccc 5160

catggagtcc ccggcccctg gaggccacag ccccgacagc cccgcagccc cctctgctcc 5220

tcctactgct gctgcccgtg ggcctcctgc tgctggctgc tgcctggtgc ctgcactggc 5280

agaggacgcg gcggaggaca ccccgccctg gggagcaggt gccccccgtc cccagtcccc 5340

aggacctgct gcttgtggag cactccggag ccacgaactt ctctctgtta aagcaagcag 5400

gagacgtgga agaaaacccc ggtcccatgc aggtctccac tgctgccctt gctgtcctcc 5460

tctgcaccat ggctctctgc aaccagttct ctgcatcact tgctgctgac acgccgaccg 5520

cctgctgctt cagctacacc tcccggcaga ttccacagaa tttcatagct gactactttg 5580

agacgagcag ccagtgctcc aagcccggtg tcatcttcct aaccaagcga agccggcagg 5640

tctgtgctga ccccagtgag gagtgggtcc agaaatatgt cagcgacctg gagctgagtg 5700

cctccggagc cacgaacttc tctctgttaa agcaagcagg agacgtggaa gaaaaccccg 5760

gtcccatgag acttctcatc ctggccctcc ttggcatctg ctctctcact gcatacattg 5820

tggaaggtgt agggagtgaa gtctcagata agaggacctg tgtgagcctc actacccagc 5880

gactgccagt tagcagaatc aagacctaca ccatcacgga aggctccttg agagcagtaa 5940

tttttattac caaacgtggc ctaaaagtct gtgctgatcc acaagccaca tgggtgagag 6000

acgtggtcag gagcatggac aggaaatcca acaccagaaa taacatgatc cagaccaagc 6060

caacaggaac ccagcaatcg accaatacag ctgtgactct gactggctag ctcgagaata 6120

aaggtacctt attttcatta gatttgtgtg ttggtttttt gtgtggcggc ctcgagatgg 6180

gagagcttga tgccaggcaa gtccaggcag gccaggcaag tccaggcagg ccaggcaagt 6240

ccaggcaggc caggcaagtc caggcaggcc aggcaagtcc aggcaggcca ggcaagtcca 6300

ggcaggccag gcaagtccag gcaggccagg caagtccagg caggccaggc aagtccaggc 6360

aggccaggca agtccaggca gggccaggca agtccaggca ggccaggcaa gtccaggcag 6420

gccaggcaag tccaggcagg atcgaattcg agctcgtacg gtgggagggc tatataagca 6480

gacctcgttt agtgaaccgt cagatcgcct ggagacgaag cttgctagcg gatccaccat 6540

gaagctactg tcttctatcg aacaagcatg cgatatttgc cgacttaaaa agctcaagtg 6600

ctccaaagaa aaaccgaagt gcgccaagtg tctgaagaac aactgggagt gtcgctactc 6660

tcccaaaacc aaaaggtctc cgctgactag ggcacatctg acagaagtgg aatcaaggct 6720

agaaagactg gaacagctat ttctactgat ttttcctcga gaagaccttg acatgatttt 6780

gaaaatggat tctttacagg atataaaagc attgttaaca ggattatttg tacaagataa 6840

tgtgaataaa gatgccgtca cagatagatt ggcttcagtg gagactgata tgcctctaac 6900

attgagacag catagaataa gtgcgacatc atcatcggaa gagagtagta acaaaggtca 6960

aagacagttg actgtatcga tgactgcaga aatggatatg gccctcatgc agggctgtgt 7020

gacctttcaa gatgtggcca tttgcttctc ccatgaagaa tggagacttc ttgatgagac 7080

tcagaggctc ctgtacctca gtgttatgct gcagaacttt gcactcataa actcccaggg 7140

ttaggattag tcatatgctg cagtgtagat ctaaataaag caatagcatc acaaataaag 7200

catttcattc actgcattct agttgtggtt tgtacgcgtg atatcaattc atctatgtcg 7260

ggtgcggaga aagaggtaat gaaatggcat cgactcgaag atctgggcgt ggttaagggt 7320

gggaaagaat atataaggtg ggggtcttat gtagttttgt atctgttttg cagcagccgc 7380

cgccgccatg agcaccaact cgtttgatgg aagcattgtg agctcatatt tgacaacgcg 7440

catgccccca tgggccgggg tgcgtcagaa tgtgatgggc tccagcattg atggtcgccc 7500

cgtcctgccc gcaaactcta ctaccttgac ctacgagacc gtgtctggaa cgccgttgga 7560

gactgcagcc tccgccgccg cttcagccgc tgcagccacc gcccgcggga ttgtgactga 7620

ctttgctttc ctgagcccgc ttgcaagcag tgcagcttcc cgttcatccg cccgcgatga 7680

caagttgacg gctcttttgg cacaattgga ttctttgacc cgggaactta atgtcgtttc 7740

tcagcagctg ttggatctgc gccagcaggt ttctgccctg aaggcttcct cccctcccaa 7800

tgcggtttaa aacataaata aaaaaccaga ctctgtttgg atttggatca agcaagtgtc 7860

ttgctgtctt tatttagggg ttttgcgcgc gcggtaggcc cgggaccagc ggtctcggtc 7920

gttgagggtc ctgtgtattt tttccaggac gtggtaaagg tgactctgga tgttcagata 7980

catgggcata agcccgtctc tggggtggag gtagcaccac tgcagagctt catgctgcgg 8040

ggtggtgttg tagatgatcc agtcgtagca ggagcgctgg gcgtggtgcc taaaaatgtc 8100

tttcagtagc aagctgattg ccaggggcag gcccttggtg taagtgttta caaagcggtt 8160

aagctgggat gggtgcatac gtggggatat gagatgcatc ttggactgta tttttaggtt 8220

ggctatgttc ccagccatat ccctccgggg attcatgttg tgcagaacca ccagcacagt 8280

gtatccggtg cacttgggaa atttgtcatg tagcttagaa ggaaatgcgt ggaagaactt 8340

ggagacgccc ttgtgacctc caagattttc catgcattcg tccataatga tggcaatggg 8400

cccacgggcg gcggcctggg cgaagatatt tctgggatca ctaacgtcat agttgtgttc 8460

caggatgaga tcgtcatagg ccatttttac aaagcgcggg cggagggtgc cagactgcgg 8520

tataatggtt ccatccggcc caggggcgta gttaccctca cagatttgca tttcccacgc 8580

tttgagttca gatgggggga tcatgtctac ctgcggggcg atgaagaaaa cggtttccgg 8640

ggtaggggag atcagctggg aagaaagcag gttcctgagc agctgcgact taccgcagcc 8700

ggtgggcccg taaatcacac ctattaccgg ctgcaactgg tagttaagag agctgcagct 8760

gccgtcatcc ctgagcaggg gggccacttc gttaagcatg tccctgactc gcatgttttc 8820

cctgaccaaa tccgccagaa ggcgctcgcc gcccagcgat agcagttctt gcaaggaagc 8880

aaagtttttc aacggtttga gaccgtccgc cgtaggcatg cttttgagcg tttgaccaag 8940

cagttccagg cggtcccaca gctcggtcac ctgctctacg gcatctcgat ccagcatatc 9000

tcctcgtttc gcgggttggg gcggctttcg ctgtacggca gtagtcggtg ctcgtccaga 9060

cgggccaggg tcatgtcttt ccacgggcgc agggtcctcg tcagcgtagt ctgggtcacg 9120

gtgaaggggt gcgctccggg ctgcgcgctg gccagggtgc gcttgaggct ggtcctgctg 9180

gtgctgaagc gctgccggtc ttcgccctgc gcgtcggcca ggtagcattt gaccatggtg 9240

tcatagtcca gcccctccgc ggcgtggccc ttggcgcgca gcttgccctt ggaggaggcg 9300

ccgcacgagg ggcagtgcag acttttgagg gcgtagagct tgggcgcgag aaataccgat 9360

tccggggagt aggcatccgc gccgcaggcc ccgcagacgg tctcgcattc cacgagccag 9420

gtgagctctg gccgttcggg gtcaaaaacc aggtttcccc catgcttttt gatgcgtttc 9480

ttacctctgg tttccatgag ccggtgtcca cgctcggtga cgaaaaggct gtccgtgtcc 9540

ccgtatacag acttgagagg cctgtcctcg agcggtgttc cgcggtcctc ctcgtataga 9600

aactcggacc actctgagac aaaggctcgc gtccaggcca gcacgaagga ggctaagtgg 9660

gaggggtagc ggtcgttgtc cactaggggg tccactcgct ccagggtgtg aagacacatg 9720

tcgccctctt cggcatcaag gaaggtgatt ggtttgtagg tgtaggccac gtgaccgggt 9780

gttcctgaag gggggctata aaagggggtg ggggcgcgtt cgtcctcact ctcttccgca 9840

tcgctgtctg cgagggccag ctgttggggt gagtactccc tctgaaaagc gggcatgact 9900

tctgcgctaa gattgtcagt ttccaaaaac gaggaggatt tgatattcac ctggcccgcg 9960

gtgatgcctt tgagggtggc cgcatccatc tggtcagaaa agacaatctt tttgttgtca 10020

agcttggtgg caaacgaccc gtagagggcg ttggacagca acttggcgat ggagcgcagg 10080

gtttggtttt tgtcgcgatc ggcgcgctcc ttggccgcga tgtttagctg cacgtattcg 10140

cgcgcaacgc accgccattc gggaaagacg gtggtgcgct cgtcgggcac caggtgcacg 10200

cgccaaccgc ggttgtgcag ggtgacaagg tcaacgctgg tggctacctc tccgcgtagg 10260

cgctcgttgg tccagcagag gcggccgccc ttgcgcgagc agaatggcgg tagggggtct 10320

agctgcgtct cgtccggggg gtctgcgtcc acggtaaaga ccccgggcag caggcgcgcg 10380

tcgaagtagt ctatcttgca tccttgcaag tctagcgcct gctgccatgc gcgggcggca 10440

agcgcgcgct cgtatgggtt gagtggggga ccccatggca tggggtgggt gagcgcggag 10500

gcgtacatgc cgcaaatgtc gtaaacgtag aggggctctc tgagtattcc aagatatgta 10560

gggtagcatc ttccaccgcg gatgctggcg cgcacgtaat cgtatagttc gtgcgaggga 10620

gcgaggaggt cgggaccgag gttgctacgg gcgggctgct ctgctcggaa gactatctgc 10680

ctgaagatgg catgtgagtt ggatgatatg gttggacgct ggaagacgtt gaagctggcg 10740

tctgtgagac ctaccgcgtc acgcacgaag gaggcgtagg agtcgcgcag cttgttgacc 10800

agctcggcgg tgacctgcac gtctagggcg cagtagtcca gggtttcctt gatgatgtca 10860

tacttatcct gtcccttttt tttccacagc tcgcggttga ggacaaactc ttcgcggtct 10920

ttccagtact cttggatcgg aaacccgtcg gcctccgaac ggtaagagcc tagcatgtag 10980

aactggttga cggcctggta ggcgcagcat cccttttcta cgggtagcgc gtatgcctgc 11040

gcggccttcc ggagcgaggt gtgggtgagc gcaaaggtgt ccctgaccat gactttgagg 11100

tactggtatt tgaagtcagt gtcgtcgcat ccgccctgct cccagagcaa aaagtccgtg 11160

cgctttttgg aacgcggatt tggcagggcg aaggtgacat cgttgaagag tatctttccc 11220

gcgcgaggca taaagttgcg tgtgatgcgg aagggtcccg gcacctcgga acggttgtta 11280

attacctggg cggcgagcac gatctcgtca aagccgttga tgttgtggcc cacaatgtaa 11340

agttccaaga agcgcgggat gcccttgatg gaaggcaatt ttttaagttc ctcgtaggtg 11400

agctcttcag gggagctgag cccgtgctct gaaagggccc agtctgcaag atgagggttg 11460

gaagcgacga atgagctcca caggtcacgg gccattagca tttgcaggtg gtcgcgaaag 11520

gtcctaaact ggcgacctat ggccattttt tctggggtga tgcagtagaa ggtaagcggg 11580

tcttgttccc agcggtccca tccaaggttc gcggctaggt ctcgcgcggc agtcactaga 11640

ggctcatctc cgccgaactt catgaccagc atgaagggca cgagctgctt cccaaaggcc 11700

cccatccaag tataggtctc tacatcgtag gtgacaaaga gacgctcggt gcgaggatgc 11760

gagccgatcg ggaagaactg gatctcccgc caccaattgg aggagtggct attgatgtgg 11820

tgaaagtaga agtccctgcg acgggccgaa cactcgtgct ggcttttgta aaaacgtgcg 11880

cagtactggc agcggtgcac gggctgtaca tcctgcacga ggttgacctg acgaccgcgc 11940

acaaggaagc agagtgggaa tttgagcccc tcgcctggcg ggtttggctg gtggtcttct 12000

acttcggctg cttgtccttg accgtctggc tgctcgaggg gagttacggt ggatcggacc 12060

accacgccgc gcgagcccaa agtccagatg tccgcgcgcg gcggtcggag cttgatgaca 12120

acatcgcgca gatgggagct gtccatggtc tggagctccc gcggcgtcag gtcaggcggg 12180

agctcctgca ggtttacctc gcatagacgg gtcagggcgc gggctagatc caggtgatac 12240

ctaatttcca ggggctggtt ggtggcggcg tcgatggctt gcaagaggcc gcatccccgc 12300

ggcgcgacta cggtaccgcg cggcgggcgg tgggccgcgg gggtgtcctt ggatgatgca 12360

tctaaaagcg gtgacgcggg cgagcccccg gaggtagggg gggctccgga cccgccggga 12420

gagggggcag gggcacgtcg gcgccgcgcg cgggcaggag ctggtgctgc gcgcgtaggt 12480

tgctggcgaa cgcgacgacg cggcggttga tctcctgaat ctggcgcctc tgcgtgaaga 12540

cgacgggccc ggtgagcttg agcctgaaag agagttcgac agaatcaatt tcggtgtcgt 12600

tgacggcggc ctggcgcaaa atctcctgca cgtctcctga gttgtcttga taggcgatct 12660

cggccatgaa ctgctcgatc tcttcctcct ggagatctcc gcgtccggct cgctccacgg 12720

tggcggcgag gtcgttggaa atgcgggcca tgagctgcga gaaggcgttg aggcctccct 12780

cgttccagac gcggctgtag accacgcccc cttcggcatc gcgggcgcgc atgaccacct 12840

gcgcgagatt gagctccacg tgccgggcga agacggcgta gtttcgcagg cgctgaaaga 12900

ggtagttgag ggtggtggcg gtgtgttctg ccacgaagaa gtacataacc cagcgtcgca 12960

acgtggattc gttgatatcc cccaaggcct caaggcgctc catggcctcg tagaagtcca 13020

cggcgaagtt gaaaaactgg gagttgcgcg ccgacacggt taactcctcc tccagaagac 13080

ggatgagctc ggcgacagtg tcgcgcacct cgcgctcaaa ggctacaggg gcctcttctt 13140

cttcttcaat ctcctcttcc ataagggcct ccccttcttc ttcttctggc ggcggtgggg 13200

gaggggggac acggcggcga cgacggcgca ccgggaggcg gtcgacaaag cgctcgatca 13260

tctccccgcg gcgacggcgc atggtctcgg tgacggcgcg gccgttctcg cgggggcgca 13320

gttggaagac gccgcccgtc atgtcccggt tatgggttgg cggggggctg ccatgcggca 13380

gggatacggc gctaacgatg catctcaaca attgttgtgt aggtactccg ccgccgaggg 13440

acctgagcga gtccgcatcg accggatcgg aaaacctctc gagaaaggcg tctaaccagt 13500

cacagtcgca aggtaggctg agcaccgtgg cgggcggcag cgggcggcgg tcggggttgt 13560

ttctggcgga ggtgctgctg atgatgtaat taaagtaggc ggtcttgaga cggcggatgg 13620

tcgacagaag caccatgtcc ttgggtccgg cctgctgaat gcgcaggcgg tcggccatgc 13680

cccaggcttc gttttgacat cggcgcaggt ctttgtagta gtcttgcatg agcctttcta 13740

ccggcacttc ttcttctcct tcctcttgtc ctgcatctct tgcatctatc gctgcggcgg 13800

cggcggagtt tggccgtagg tggcgccctc ttcctcccat gcgtgtgacc ccgaagcccc 13860

tcatcggctg aagcagggct aggtcggcga caacgcgctc ggctaatatg gcctgctgca 13920

cctgcgtgag ggtagactgg aagtcatcca tgtccacaaa gcggtggtat gcgcccgtgt 13980

tgatggtgta agtgcagttg gccataacgg accagttaac ggtctggtga cccggctgcg 14040

agagctcggt gtacctgaga cgcgagtaag ccctcgagtc aaatacgtag tcgttgcaag 14100

tccgcaccag gtactggtat cccaccaaaa agtgcggcgg cggctggcgg tagaggggcc 14160

agcgtagggt ggccggggct ccgggggcga gatcttccaa cataaggcga tgatatccgt 14220

agatgtacct ggacatccag gtgatgccgg cggcggtggt ggaggcgcgc ggaaagtcgc 14280

ggacgcggtt ccagatgttg cgcagcggca aaaagtgctc catggtcggg acgctctggc 14340

cggtcaggcg cgcgcaatcg ttgacgctct agcgtgcaaa aggagagcct gtaagcgggc 14400

actcttccgt ggtctggtgg ataaattcgc aagggtatca tggcggacga ccggggttcg 14460

agccccgtat ccggccgtcc gccgtgatcc atgcggttac cgcccgcgtg tcgaacccag 14520

gtgtgcgacg tcagacaacg ggggagtgct ccttttggct tccttccagg cgcggcggct 14580

gctgcgctag cttttttggc cactggccgc gcgcagcgta agcggttagg ctggaaagcg 14640

aaagcattaa gtggctcgct ccctgtagcc ggagggttat tttccaaggg ttgagtcgcg 14700

ggacccccgg ttcgagtctc ggaccggccg gactgcggcg aacgggggtt tgcctccccg 14760

tcatgcaaga ccccgcttgc aaattcctcc ggaaacaggg acgagcccct tttttgcttt 14820

tcccagatgc atccggtgct gcggcagatg cgcccccctc ctcagcagcg gcaagagcaa 14880

gagcagcggc agacatgcag ggcaccctcc cctcctccta ccgcgtcagg aggggcgaca 14940

tccgcggttg acgcggcagc agatggtgat tacgaacccc cgcggcgccg ggcccggcac 15000

tacctggact tggaggaggg cgagggcctg gcgcggctag gagcgccctc tcctgagcgg 15060

cacccaaggg tgcagctgaa gcgtgatacg cgtgaggcgt acgtgccgcg gcagaacctg 15120

tttcgcgacc gcgagggaga ggagcccgag gagatgcggg atcgaaagtt ccacgcaggg 15180

cgcgagctgc ggcatggcct gaatcgcgag cggttgctgc gcgaggagga ctttgagccc 15240

gacgcgcgaa ccgggattag tcccgcgcgc gcacacgtgg cggccgccga cctggtaacc 15300

gcatacgagc agacggtgaa ccaggagatt aactttcaaa aaagctttaa caaccacgtg 15360

cgtacgcttg tggcgcgcga ggaggtggct ataggactga tgcatctgtg ggactttgta 15420

agcgcgctgg agcaaaaccc aaatagcaag ccgctcatgg cgcagctgtt ccttatagtg 15480

cagcacagca gggacaacga ggcattcagg gatgcgctgc taaacatagt agagcccgag 15540

ggccgctggc tgctcgattt gataaacatc ctgcagagca tagtggtgca ggagcgcagc 15600

ttgagcctgg ctgacaaggt ggccgccatc aactattcca tgcttagcct gggcaagttt 15660

tacgcccgca agatatacca taccccttac gttcccatag acaaggaggt aaagatcgag 15720

gggttctaca tgcgcatggc gctgaaggtg cttaccttga gcgacgacct gggcgtttat 15780

cgcaacgagc gcatccacaa ggccgtgagc gtgagccggc ggcgcgagct cagcgaccgc 15840

gagctgatgc acagcctgca aagggccctg gctggcacgg gcagcggcga tagagaggcc 15900
gagtcctact ttgacgcggg cgctgacctg cgctgggccc caagccgacg cgccctggag 15960
gcagctgggg ccggacctgg gctggcggtg gcacccgcgc gcgctggcaa cgtcggcggc 16020
gtggaggaat atgacgagga cgatgagtac gagccagagg acggcgagta ctaagcggtg 16080
atgtttctga tcagatgatg caagacgcaa cggacccggc ggtgcgggcg gcgctgcaga 16140
gccagccgtc cggccttaac tccacggacg actggcgcca ggtcatggac cgcatcatgt 16200
cgctgactgc gcgcaatcct gacgcgttcc ggcagcagcc gcaggccaac cggctctccg 16260
caattctgga agcggtggtc ccggcgcgcg caaaccccac gcacgagaag gtgctggcga 16320
tcgtaaacgc gctggccgaa aacagggcca tccggcccga cgaggccggc ctggtctacg 16380
acgcgctgct tcagcgcgtg gctcgttaca acagcggcaa cgtgcagacc aacctggacc 16440
ggctggtggg ggatgtgcgc gaggccgtgg cgcagcgtga gcgcgcgcag cagcagggca 16500
acctgggctc catggttgca ctaaacgcct tcctgagtac acagcccgcc aacgtgccgc 16560
ggggacagga ggactacacc aactttgtga gcgcactgcg gctaatggtg actgagacac 16620
cgcaaagtga ggtgtaccag tctgggccag actatttttt ccagaccagt agacaaggcc 16680
tgcagaccgt aaacctgagc caggctttca aaaacttgca ggggctgtgg ggggtgcggg 16740
ctcccacagg cgaccgcgcg accgtgtcta gcttgctgac gcccaactcg cgcctgttgc 16800
tgctgctaat agcgcccttc acggacagtg gcagcgtgtc ccgggacaca tacctaggtc 16860
acttgctgac actgtaccgc gaggccatag gtcaggcgca tgtggacgag catactttcc 16920
aggagattac aagtgtcagc cgcgcgctgg ggcaggagga cacgggcagc ctggaggcaa 16980
ccctaaacta cctgctgacc aaccggcggc agaagatccc ctcgttgcac agtttaaaca 17040
gcgaggagga gcgcattttg cgctacgtgc agcagagcgt gagccttaac ctgatgcgcg 17100
acggggtaac gcccagcgtg gcgctggaca tgaccgcgcg caacatggaa ccgggcatgt 17160
atgcctcaaa ccggccgttt atcaaccgcc taatggacta cttgcatcgc gcggccgccg 17220
tgaaccccga gtatttcacc aatgccatct tgaacccgca ctggctaccg ccccctggtt 17280
tctacaccgg gggattcgag gtgcccgagg gtaacgatgg attcctctgg gacgacatag 17340
acgacagcgt gttttccccg caaccgcaga ccctgctaga gttgcaacag cgcgagcagg 17400
cagaggcggc gctgcgaaag gaaagcttcc gcaggccaag cagcttgtcc gatctaggcg 17460
ctgcggcccc gcggtcagat gctagtagcc catttccaag cttgataggg tctcttacca 17520
gcactcgcac cacccgcccg cgcctgctgg gcgaggagga gtacctaaac aactcgctgc 17580
tgcagccgca gcgcgaaaaa aacctgcctc cggcatttcc caacaacggg atagagagcc 17640
tagtggacaa gatgagtaga tggaagacgt acgcgcagga gcacagggac gtgccaggcc 17700

cgcgcccgcc cacccgtcgt caaaggcacg accgtcagcg gggtctggtg tgggaggacg 17760

atgactcggc agacgacagc agcgtcctgg atttgggagg gagtggcaac ccgtttgcgc 17820

accttcgccc caggctgggg agaatgtttt aaaaaaaaaa aaagcatgat gcaaaataaa 17880

aaactcacca aggccatggc accgagcgtt ggttttcttg tattcccctt agtatgcggc 17940

gcgcggcgat gtatgaggaa ggtcctcctc cctcctacga gagtgtggtg agcgcggcgc 18000

cagtggcggc ggcgctgggt tctcccttcg atgctcccct ggacccgccg tttgtgcctc 18060

cgcggtacct gcggcctacc ggggggagaa acagcatccg ttactctgag ttggcacccc 18120

tattcgacac cacccgtgtg tacctggtgg acaacaagtc aacggatgtg gcatccctga 18180

actaccagaa cgaccacagc aactttctga ccacggtcat tcaaaacaat gactacagcc 18240

cgggggaggc aagcacacag accatcaatc ttgacgaccg gtcgcactgg ggcggcgacc 18300

tgaaaaccat cctgcatacc aacatgccaa atgtgaacga gttcatgttt accaataagt 18360

ttaaggcgcg ggtgatggtg tcgcgcttgc ctactaagga caatcaggtg gagctgaaat 18420

acgagtgggt ggagttcacg ctgcccgagg gcaactactc cgagaccatg accatagacc 18480

ttatgaacaa cgcgatcgtg gagcactact tgaaagtggg cagacagaac ggggttctgg 18540

aaagcgacat cggggtaaag tttgacaccc gcaacttcag actggggttt gaccccgtca 18600

ctggtcttgt catgcctggg gtatatacaa acgaagcctt ccatccagac atcattttgc 18660

tgccaggatg cggggtggac ttcacccaca gccgcctgag caacttgttg ggcatccgca 18720

agcggcaacc cttccaggag ggctttagga tcacctacga tgatctggag ggtggtaaca 18780

ttcccgcact gttggatgtg gacgcctacc aggcgagctt gaaagatgac accgaacagg 18840

gcgggggtgg cgcaggcggc agcaacagca gtggcagcgg cgcggaagag aactccaacg 18900

cggcagccgc ggcaatgcag ccggtggagg acatgaacga tcatgccatt cgcggcgaca 18960

cctttgccac acgggctgag gagaagcgcg ctgaggccga agcagcggcc gaagctgccg 19020

cccccgctgc gcaacccgag gtcgagaagc ctcagaagaa accggtgatc aaacccctga 19080

cagaggacag caagaaacgc agttacaacc taataagcaa tgacagcacc ttcacccagt 19140

accgcagctg gtaccttgca tacaactacg gcgaccctca gaccggaatc cgctcatgga 19200

ccctgctttg cactcctgac gtaacctgcg gctcggagca ggtctactgg tcgttgccag 19260

acatgatgca agaccccgtg accttccgct ccacgcgcca gatcagcaac tttccggtgg 19320

tgggcgccga gctgttgccc gtgcactcca agagcttcta caacgaccag gccgtctact 19380

cccaactcat ccgccagttt acctctctga cccacgtgtt caatcgcttt cccgagaacc 19440

agattttggc gcgcccgcca gcccccacca tcaccaccgt cagtgaaaac gttcctgctc 19500

tcacagatca cgggacgcta ccgctgcgca acagcatcgg aggagtccag cgagtgacca 19560

ttactgacgc cagacgccgc acctgcccct acgtttacaa ggccctgggc atagtctcgc 19620

cgcgcgtcct atcgagccgc actttttgag caagcatgtc catccttata tcgcccagca 19680

ataacacagg ctggggcctg cgcttcccaa gcaagatgtt tggcggggcc aagaagcgct 19740

ccgaccaaca cccagtgcgc gtgcgcgggc actaccgcgc gccctggggc gcgcacaaac 19800

gcggccgcac tgggcgcacc accgtcgatg acgccatcga cgcggtggtg gaggaggcgc 19860

gcaactacac gcccacgccg ccaccagtgt ccacagtgga cgcggccatt cagaccgtgg 19920

tgcgcggagc ccggcgctat gctaaaatga agagacggcg gaggcgcgta gcacgtcgcc 19980

accgccgccg acccggcact gccgcccaac gcgcggcggc ggccctgctt aaccgcgcac 20040

gtcgcaccgg ccgacgggcg gccatgcggg ccgctcgaag gctggccgcg ggtattgtca 20100

ctgtgccccc caggtccagg cgacgagcgg ccgccgcagc agccgcggcc attagtgcta 20160

tgactcaggg tcgcaggggc aacgtgtatt gggtgcgcga ctcggttagc ggcctgcgcg 20220

tgcccgtgcg cacccgcccc ccgcgcaact agattgcaag aaaaaactac ttagactcgt 20280

actgttgtat gtatccagcg gcggcggcgc gcaacgaagc tatgtccaag cgcaaaatca 20340

aagaagagat gctccaggtc atcgcgccgg agatctatgg ccccccgaag aaggaagagc 20400

aggattacaa gccccgaaag ctaaagcggg tcaaaaagaa aaagaaagat gatgatgatg 20460

aacttgacga cgaggtggaa ctgctgcacg ctaccgcgcc caggcgacgg gtacagtgga 20520

aaggtcgacg cgtaaaacgt gttttgcgac ccggcaccac cgtagtcttt acgcccggtg 20580

agcgctccac ccgcacctac aagcgcgtgt atgatgaggt gtacggcgac gaggacctgc 20640

ttgagcaggc caacgagcgc ctcggggagt ttgcctacgg aaagcggcat aaggacatgc 20700

tggcgttgcc gctggacgag ggcaacccaa cacctagcct aaagcccgta acactgcagc 20760

aggtgctgcc cgcgcttgca ccgtccgaag aaaagcgcgg cctaaagcgc gagtctggtg 20820

acttggcacc caccgtgcag ctgatggtac ccaagcgcca gcgactggaa gatgtcttgg 20880

aaaaaatgac cgtggaacct gggctggagc ccgaggtccg cgtgcggcca atcaagcagg 20940

tggcgccggg actgggcgtg cagaccgtgg acgttcagat acccactacc agtagcacca 21000

gtattgccac cgccacagag ggcatggaga cacaaacgtc cccggttgcc tcagcggtgg 21060

cggatgccgc ggtgcaggcg gtcgctgcgg ccgcgtccaa gacctctacg gaggtgcaaa 21120

cggacccgtg gatgtttcgc gtttcagccc cccggcgccc gcgccgttcg aggaagtacg 21180

gcgccgccag cgcgctactg cccgaatatg ccctacatcc ttccattgcg cctacccccg 21240

gctatcgtgg ctacacctac cgccccagaa gacgagcaac tacccgacgc cgaaccacca 21300

ctggaacccg ccgccgccgt cgccgtcgcc agcccgtgct ggccccgatt tccgtgcgca 21360

gggtggctcg cgaaggaggc aggaccctgg tgctgccaac agcgcgctac caccccagca 21420

tcgtttaaaa gccggtcttt gtggttcttg cagatatggc cctcacctgc cgcctccgtt 21480

tcccggtgcc gggattccga ggaagaatgc accgtaggag gggcatggcc ggccacggcc 21540

tgacgggcgg catgcgtcgt gcgcaccacc ggcggcggcg cgcgtcgcac cgtcgcatgc 21600

gcggcggtat cctgcccctc cttattccac tgatcgccgc ggcgattggc gccgtgcccg 21660

gaattgcatc cgtggccttg caggcgcaga gacactgatt aaaaacaagt tgcatgtgga 21720

aaaatcaaaa taaaaagtct ggactctcac gctcgcttgg tcctgtaact attttgtaga 21780

atggaagaca tcaactttgc gtctctggcc ccgcgacacg gctcgcgccc gttcatggga 21840

aactggcaag atatcggcac cagcaatatg agcggtggcg ccttcagctg gggctcgctg 21900

tggagcggca ttaaaaattt cggttccacc gttaagaact atggcagcaa ggcctggaac 21960

agcagcacag gccagatgct gagggataag ttgaaagagc aaaatttcca acaaaaggtg 22020

gtagatggcc tggcctctgg cattagcggg gtggtggacc tggccaacca ggcagtgcaa 22080

aataagatta acagtaagct tgatccccgc cctcccgtag aggagcctcc accggccgtg 22140

gagacagtgt ctccagaggg gcgtggcgaa aagcgtccgc gccccgacag ggaagaaact 22200

ctggtgacgc aaatagacga gcctccctcg tacgaggagg cactaaagca aggcctgccc 22260

accacccgtc ccatcgcgcc catggctacc ggagtgctgg gccagcacac acccgtaacg 22320

ctggacctgc ctccccccgc cgacacccag cagaaacctg tgctgccagg cccgaccgcc 22380

gttgttgtaa cccgtcctag ccgcgcgtcc ctgcgccgcg ccgccagcgg tccgcgatcg 22440

ttgcggcccg tagccagtgg caactggcaa agcacactga acagcatcgt gggtctgggg 22500

gtgcaatccc tgaagcgccg acgatgcttc tgatagctaa cgtgtcgtat gtgtgtcatg 22560

tatgcgtcca tgtcgccgcc agaggagctg ctgagccgcc gcgcgcccgc tttccaagat 22620

ggctacccct tcgatgatgc cgcagtggtc ttacatgcac atctcgggcc aggacgcctc 22680

ggagtacctg agccccgggc tggtgcagtt tgcccgcgcc accgagacgt acttcagcct 22740

gaataacaag tttagaaacc ccacggtggc gcctacgcac gacgtgacca cagaccggtc 22800

ccagcgtttg acgctgcggt tcatccctgt ggaccgtgag gatactgcgt actcgtacaa 22860

ggcgcggttc accctagctg tgggtgataa ccgtgtgctg gacatggctt ccacgtactt 22920

tgacatccgc ggcgtgctgg acaggggccc tacttttaag ccctactctg gcactgccta 22980

caacgccctg gctcccaagg gtgccccaaa tccttgcgaa tgggatgaag ctgctactgc 23040

tcttgaaata aacctagaag aagaggacga tgacaacgaa gacgaagtag acgagcaagc 23100

tgagcagcaa aaaactcacg tatttgggca ggcgccttat tctggtataa atattacaaa 23160

ggagggtatt caaataggtg tcgaaggtca aacacctaaa tatgccgata aaacatttca 23220

acctgaacct caaataggag aatctcagtg gtacgaaaca gaaattaatc atgcagctgg 23280

gagagtccta aaaaagacta ccccaatgaa accatgttac ggttcatatg caaaacccac 23340

aaatgaaaat ggagggcaag gcattcttgt aaagcaacaa aatggaaagc tagaaagtca 23400

agtggaaatg caatttttct caactactga ggcagccgca ggcaatggtg ataacttgac 23460

tcctaaagtg gtattgtaca gtgaagatgt agatatagaa accccagaca ctcatatttc 23520

ttacatgccc actattaagg aaggtaactc acgagaacta atgggccaac aatctatgcc 23580

caacaggcct aattacattg cttttaggga caattttatt ggtctaatgt attacaacag 23640

cacgggtaat atgggtgttc tggcgggcca agcatcgcag ttgaatgctg ttgtagattt 23700

gcaagacaga aacacagagc tttcatacca gcttttgctt gattccattg gtgatagaac 23760

caggtacttt tctatgtgga atcaggctgt tgacagctat gatccagatg ttagaattat 23820

tgaaaatcat ggaactgaag atgaacttcc aaattactgc tttccactgg gaggtgtgat 23880

taatacagag actcttacca aggtaaaacc taaaacaggt caggaaaatg gatgggaaaa 23940

agatgctaca gaattttcag ataaaaatga aataagagtt ggaaataatt ttgccatgga 24000

aatcaatcta aatgccaacc tgtggagaaa tttcctgtac tccaacatag cgctgtattt 24060

gcccgacaag ctaaagtaca gtccttccaa cgtaaaaatt tctgataacc caaacaccta 24120

cgactacatg aacaagcgag tggtggctcc cgggctagtg gactgctaca ttaaccttgg 24180

agcacgctgg tcccttgact atatggacaa cgtcaaccca tttaaccacc accgcaatgc 24240

tggcctgcgc taccgctcaa tgttgctggg caatggtcgc tatgtgccct tccacatcca 24300

ggtgcctcag aagttctttg ccattaaaaa cctccttctc ctgccgggct catacaccta 24360

cgagtggaac ttcaggaagg atgttaacat ggttctgcag agctccctag gaaatgacct 24420

aagggttgac ggagccagca ttaagtttga tagcatttgc ctttacgcca ccttcttccc 24480

catggcccac aacaccgcct ccacgcttga ggccatgctt agaaacgaca ccaacgacca 24540

gtcctttaac gactatctct ccgccgccaa catgctctac cctatacccg ccaacgctac 24600

caacgtgccc atatccatcc cctcccgcaa ctgggcggct ttccgcggct gggccttcac 24660

gcgccttaag actaaggaaa ccccatcact gggctcgggc tacgaccctt attacaccta 24720

ctctggctct ataccctacc tagatggaac cttttacctc aaccacacct ttaagaaggt 24780

ggccattacc tttgactctt ctgtcagctg gcctggcaat gaccgcctgc ttacccccaa 24840

cgagtttgaa attaagcgct cagttgacgg ggagggttac aacgttgccc agtgtaacat 24900

gaccaaagac tggttcctgg tacaaatgct agctaactat aacattggct accagggctt 24960

ctatatccca gagagctaca aggaccgcat gtactccttc tttagaaact tccagcccat 25020

gagccgtcag gtggtggatg atactaaata caaggactac caacaggtgg gcatcctaca 25080

ccaacacaac aactctggat ttgttggcta ccttgccccc accatgcgcg aaggacaggc 25140

ctaccctgct aacttcccct atccgcttat aggcaagacc gcagttgaca gcattaccca 25200

gaaaaagttt ctttgcgatc gcaccctttg gcgcatccca ttctccagta actttatgtc 25260

catgggcgca ctcacagacc tgggccaaaa ccttctctac gccaactccg cccacgcgct 25320

agacatgact tttgaggtgg atcccatgga cgagcccacc cttctttatg ttttgtttga 25380

agtctttgac gtggtccgtg tgcaccagcc gcaccgcggc gtcatcgaaa ccgtgtacct 25440

gcgcacgccc ttctcggccg gcaacgccac aacataaaga agcaagcaac atcaacaaca 25500

gctgccgcca tgggctccag tgagcaggaa ctgaaagcca ttgtcaaaga tcttggttgt 25560

gggccatatt ttttgggcac ctatgacaag cgctttccag gctttgtttc tccacacaag 25620

ctcgcctgcg ccatagtcaa tacggccggt cgcgagactg ggggcgtaca ctggatggcc 25680

tttgcctgga acccgcactc aaaaacatgc tacctctttg agccctttgg cttttctgac 25740

cagcgactca agcaggttta ccagtttgag tacgagtcac tcctgcgccg tagcgccatt 25800

gcttcttccc ccgaccgctg tataacgctg gaaaagtcca cccaaagcgt acaggggccc 25860

aactcggccg cctgtggact attctgctgc atgtttctcc acgcctttgc caactggccc 25920

caaactccca tggatcacaa ccccaccatg aaccttatta ccggggtacc caactccatg 25980

ctcaacagtc cccaggtaca gcccaccctg cgtcgcaacc aggaacagct ctacagcttc 26040

ctggagcgcc actcgcccta cttccgcagc cacagtgcgc agattaggag cgccacttct 26100

ttttgtcact tgaaaaacat gtaaaaataa tgtactagag acactttcaa taaaggcaaa 26160

tgcttttatt tgtacactct cgggtgatta tttaccccca cccttgccgt ctgcgccgtt 26220

taaaaatcaa aggggttctg ccgcgcatcg ctatgcgcca ctggcaggga cacgttgcga 26280

tactggtgtt tagtgctcca cttaaactca ggcacaacca tccgcggcag ctcggtgaag 26340

ttttcactcc acaggctgcg caccatcacc aacgcgttta gcaggtcggg cgccgatatc 26400

ttgaagtcgc agttggggcc tccgccctgc gcgcgcgagt tgcgatacac agggttgcag 26460

cactggaaca ctatcagcgc cgggtggtgc acgctggcca gcacgctctt gtcggagatc 26520

agatccgcgt ccaggtcctc cgcgttgctc agggcgaacg gagtcaactt tggtagctgc 26580

cttcccaaaa agggcgcgtg cccaggcttt gagttgcact cgcaccgtag tggcatcaaa 26640

aggtgaccgt gcccggtctg ggcgttagga tacagcgcct gcataaaagc cttgatctgc 26700

ttaaaagcca cctgagcctt tgcgccttca gagaagaaca tgccgcaaga cttgccggaa 26760

aactgattgg ccggacaggc cgcgtcgtgc acgcagcacc ttgcgtcggt gttggagatc 26820

tgcaccacat ttcggcccca ccggttcttc acgatcttgg ccttgctaga ctgctccttc 26880

agcgcgcgct gcccgttttc gctcgtcaca tccatttcaa tcacgtgctc cttatttatc 26940

ataatgcttc cgtgtagaca cttaagctcg ccttcgatct cagcgcagcg gtgcagccac 27000

aacgcgcagc ccgtgggctc gtgatgcttg taggtcacct ctgcaaacga ctgcaggtac 27060

gcctgcagga atcgccccat catcgtcaca aaggtcttgt tgctggtgaa ggtcagctgc 27120

aacccgcggt gctcctcgtt cagccaggtc ttgcatacgg ccgccagagc ttccacttgg 27180

tcaggcagta gtttgaagtt cgcctttaga tcgttatcca cgtggtactt gtccatcagc 27240

gcgcgcgcag cctccatgcc cttctcccac gcagacacga tcggcacact cagcgggttc 27300

atcaccgtaa tttcactttc cgcttcgctg ggctcttcct cttcctcttg cgtccgcata 27360

ccacgcgcca ctgggtcgtc ttcattcagc cgccgcactg tgcgcttacc tcctttgcca 27420

tgcttgatta gcaccggtgg gttgctgaaa cccaccattt gtagcgccac atcttctctt 27480

tcttcctcgc tgtccacgat tacctctggt gatggcgggc gctcgggctt gggagaaggg 27540

cgcttctttt tcttcttggg cgcaatggcc aaatccgccg ccgaggtcga tggccgcggg 27600

ctgggtgtgc gcggcaccag cgcgtcttgt gatgagtctt cctcgtcctc ggactcgata 27660

cgccgcctca tccgcttttt tgggggcgcc cggggaggcg gcggcgacgg ggacggggac 27720

gacacgtcct ccatggttgg gggacgtcgc gccgcaccgc gtccgcgctc gggggtggtt 27780

tcgcgctgct cctcttcccg actggccatt tccttctcct ataggcagaa aaagatcatg 27840

gagtcagtcg agaagaagga cagcctaacc gccccctctg agttcgccac caccgcctcc 27900

accgatgccg ccaacgcgcc taccaccttc cccgtcgagg cacccccgct tgaggaggag 27960

gaagtgatta tcgagcagga cccaggtttt gtaagcgaag acgacgagga ccgctcagta 28020

ccaacagagg ataaaaagca agaccaggac aacgcagagg caaacgagga acaagtcggg 28080

cggggggacg aaaggcatgg cgactaccta gatgtgggag acgacgtgct gttgaagcat 28140

ctgcagcgcc agtgcgccat tatctgcgac gcgttgcaag agcgcagcga tgtgcccctc 28200

gccatagcgg atgtcagcct tgcctacgaa cgccacctat tctcaccgcg cgtacccccc 28260

aaacgccaag aaaacggcac atgcgagccc aacccgcgcc tcaacttcta ccccgtattt 28320

gccgtgccag aggtgcttgc cacctatcac atctttttcc aaaactgcaa gataccccta 28380

tcctgccgtg ccaaccgcag ccgagcggac aagcagctgg ccttgcggca gggcgctgtc 28440

atacctgata tcgcctcgct caacgaagtg ccaaaaatct ttgagggtct tggacgcgac 28500

gagaagcgcg cggcaaacgc tctgcaacag gaaaacagcg aaaatgaaag tcactctgga 28560

gtgttggtgg aactcgaggg tgacaacgcg cgcctagccg tactaaaacg cagcatcgag 28620

gtcacccact ttgcctaccc ggcacttaac ctacccccca aggtcatgag cacagtcatg 28680

agtgagctga tcgtgcgccg tgcgcagccc ctggagaggg atgcaaattt gcaagaacaa 28740

acagaggagg gcctacccgc agttggcgac gagcagctag cgcgctggct tcaaacgcgc 28800

gagcctgccg acttggagga gcgacgcaaa ctaatgatgg ccgcagtgct cgttaccgtg 28860

gagcttgagt gcatgcagcg gttctttgct gacccggaga tgcagcgcaa gctagaggaa 28920

acattgcact acacctttcg acagggctac gtacgccagg cctgcaagat ctccaacgtg 28980

gagctctgca acctggtctc ctaccttgga attttgcacg aaaaccgcct tgggcaaaac 29040

gtgcttcatt ccacgctcaa gggcgaggcg cgccgcgact acgtccgcga ctgcgtttac 29100

ttatttctat gctacacctg gcagacggcc atgggcgttt ggcagcagtg cttggaggag 29160

tgcaacctca aggagctgca gaaactgcta aagcaaaact tgaaggacct atggacggcc 29220

ttcaacgagc gctccgtggc cgcgcacctg gcggacatca ttttccccga acgcctgctt 29280

aaaaccctgc aacagggtct gccagacttc accagtcaaa gcatgttgca gaactttagg 29340

aactttatcc tagagcgctc aggaatcttg cccgccacct gctgtgcact tcctagcgac 29400

tttgtgccca ttaagtaccg cgaatgccct ccgccgcttt ggggccactg ctaccttctg 29460

cagctagcca actaccttgc ctaccactct gacataatgg aagacgtgag cggtgacggt 29520

ctactggagt gtcactgtcg ctgcaaccta tgcaccccgc accgctccct ggtttgcaat 29580

tcgcagctgc ttaacgaaag tcaaattatc ggtacctttg agctgcaggg tccctcgcct 29640

gacgaaaagt ccgcggctcc ggggttgaaa ctcactccgg ggctgtggac gtcggcttac 29700

cttcgcaaat ttgtacctga ggactaccac gcccacgaga ttaggttcta cgaagaccaa 29760

tcccgcccgc ctaatgcgga gcttaccgcc tgcgtcatta cccagggcca cattcttggc 29820

caattgcaag ccatcaacaa agcccgccaa gagtttctgc tacgaaaggg acggggggtt 29880

tacttggacc cccagtccgg cgaggagctc aacccaatcc ccccgccgcc gcagccctat 29940

cagcagcagc cgcgggccct tgcttcccag gatggcaccc aaaaagaagc tgcagctgcc 30000

gccgccaccc acggacgagg aggaatactg ggacagtcag gcagaggagg ttttggacga 30060

ggaggaggag gacatgatgg aagactggga gagcctagac gaggaagctt ccgaggtcga 30120

agaggtgtca gacgaaacac cgtcaccctc ggtcgcattc ccctcgccgg cgccccagaa 30180

atcggcaacc ggttccagca tggctacaac ctccgctcct caggcgccgc cggcactgcc 30240

cgttcgccga cccaaccgta gatgggacac cactggaacc agggccggta agtccaagca 30300

gccgccgccg ttagcccaag agcaacaaca gcgccaaggc taccgctcat ggcgcgggca 30360

caagaacgcc atagttgctt gcttgcaaga ctgtgggggc aacatctcct tcgcccgccg 30420

ctttcttctc taccatcacg gcgtggcctt cccccgtaac atcctgcatt actaccgtca 30480

tctctacagc ccatactgca ccggcggcag cggcagcggc agcaacagca gcggccacac 30540

agaagcaaag gcgaccggat agcaagactc tgacaaagcc caagaaatcc acagcggcgg 30600

cagcagcagg aggaggagcg ctgcgtctgg cgcccaacga acccgtatcg acccgcgagc 30660

ttagaaacag gatttttccc actctgtatg ctatatttca acagagcagg ggccaagaac 30720

aagagctgaa aataaaaaac aggtctctgc gatccctcac ccgcagctgc ctgtatcaca 30780

aaagcgaaga tcagcttcgg cgcacgctgg aagacgcgga ggctctcttc agtaaatact 30840

gcgcgctgac tcttaaggac tagtttcgcg ccctttctca aatttaagcg cgaaaactac 30900

gtcatctcca gcggccacac ccggcgccag cacctgtcgt cagcgccatt atgagcaagg 30960

aaattcccac gccctacatg tggagttacc agccacaaat gggacttgcg gctggagctg 31020

cccaagacta ctcaacccga ataaactaca tgagcgcggg accccacatg atatcccggg 31080

tcaacggaat acgcgcccac cgaaaccgaa ttctcttgga acaggcggct attaccacca 31140

cacctcgtaa taaccttaat ccccgtagtt ggcccgctgc cctggtgtac caggaaagtc 31200

ccgctcccac cactgtggta cttcccagag acgcccaggc cgaagttcag atgactaact 31260

caggggcgca gcttgcgggc ggctttcgtc acagggtgcg gtcgcccggg cagggtataa 31320

ctcacctgac aatcagaggg cgaggtattc agctcaacga cgagtcggtg agctcctcgc 31380

ttggtctccg tccggacggg acatttcaga tcggcggcgc cggccgctct tcattcacgc 31440

ctcgtcaggc aatcctaact ctgcagacct cgtcctctga gccgcgctct ggaggcattg 31500

gaactctgca atttattgag gagtttgtgc catcggtcta ctttaacccc ttctcgggac 31560

ctcccggcca ctatccggat caatttattc ctaactttga cgcggtaaag gactcggcgg 31620

acggctacga ctgaatgtta agtggagagg cagagcaact gcgcctgaaa cacctggtcc 31680

actgtcgccg ccacaagtgc tttgcccgcg actccggtga gttttgctac tttgaattgc 31740

ccgaggatca tatcgagggc ccggcgcacg gcgtccggct taccgcccag ggagagcttg 31800

cccgtagcct gattcgggag tttacccagc gccccctgct agttgagcgg gacaggggac 31860

cctgtgttct cactgtgatt tgcaactgtc ctaaccctgg attacatcaa gatcctctag 31920

ttataactag agtacccggg gatcttattc cctttaacta ataaaaaaaa ataataaagc 31980

atcacttact taaaatcagt tagcaaattt ctgtccagtt tattcagcag cacctccttg 32040

ccctcctccc agctctggta ttgcagcttc ctcctggctg caaactttct ccacaatcta 32100

aatggaatgt cagtttcctc ctgttcctgt ccatccgcac ccactatctt catgttgttg 32160

cagatgaagc gcgcaagacc gtctgaagat accttcaacc ccgtgtatcc atatgacacg 32220

gaaaccggtc ctccaactgt gccttttctt actcctccct ttgtatcccc caatgggttt 32280

caagagagtc cccctggggt actctctttg cgcctatccg aacctctagt tacctccaat 32340

ggcatgcttg cgctcaaaat gggcaacggc ctctctctgg acgaggccgg caaccttacc 32400

tcccaaaatg taaccactgt gagcccacct ctcaaaaaaa ccaagtcaaa cataaacctg 32460

gaaatatctg cacccctcac agttacctca gaagccctaa ctgtggctgc cgccgcacct 32520

ctaatggtcg cgggcaacac actcaccatg caatcacagg ccccgctaac cgtgcacgac 32580

tccaaactta gcattgccac ccaaggaccc ctcacagtgt cagaaggaaa gctagccctg 32640

caaacatcag gccccctcac caccaccgat agcagtaccc ttactatcac tgcctcaccc 32700

cctctaacta ctgccactgg tagcttgggc attgacttga aagagcccat ttatacacaa 32760

aatggaaaac taggactaaa gtacggggct cctttgcatg taacagacga cctaaacact 32820

ttgaccgtag caactggtcc aggtgtgact attaataata cttccttgca aactaaagtt 32880

actggagcct tgggttttga ttcacaaggc aatatgcaac ttaatgtagc aggaggacta 32940

aggattgatt ctcaaaacag acgccttata cttgatgtta gttatccgtt tgatgctcaa 33000

aaccaactaa atctaagact aggacagggc cctcttttta taaactcagc ccacaacttg 33060

gatattaact acaacaaagg cctttacttg tttacagctt caaacaattc caaaaagctt 33120

gaggttaacc taagcactgc caaggggttg atgtttgacg ctacagccat agccattaat 33180

gcaggagatg ggcttgaatt tggttcacct aatgcaccaa acacaaatcc cctcaaaaca 33240

aaaattggcc atggcctaga atttgattca aacaaggcta tggttcctaa actaggaact 33300

ggccttagtt ttgacagcac aggtgccatt acagtaggaa acaaaaataa tgataagcta 33360

actttgtgga ccacaccagc tccatctcct aactgtagac taaatgcaga gaaagatgct 33420

aaactcactt tggtcttaac aaaatgtggc agtcaaatac ttgctacagt ttcagttttg 33480

gctgttaaag gcagtttggc tccaatatct ggaacagttc aaagtgctca tcttattata 33540

agatttgacg aaaatggagt gctactaaac aattccttcc tggacccaga atattggaac 33600

tttagaaatg gagatcttac tgaaggcaca gcctatacaa acgctgttgg atttatgcct 33660

aacctatcag cttatccaaa atctcacggt aaaactgcca aaagtaacat tgtcagtcaa 33720

gtttacttaa acggagacaa aactaaacct gtaacactaa ccattacact aaacggtaca 33780

caggaaacag gagacacaac tccaagtgca tactctatgt cattttcatg ggactggtct 33840

ggccacaact acattaatga aatatttgcc acatcctctt acactttttc atacattgcc 33900

caagaataaa gaatcgtttg tgttatgttt caacgtgttt atttttcaat tgcagaaaat 33960

ttcaagtcat ttttcattca gtagtatagc cccaccacca catagcttat acagatcacc 34020

gtaccttaat caaactcaca gaaccctagt attcaacctg ccacctccct cccaacacac 34080

agagtacaca gtcctttctc cccggctggc cttaaaaagc atcatatcat gggtaacaga 34140

catattctta ggtgttatat tccacacggt ttcctgtcga gccaaacgct catcagtgat 34200

attaataaac tccccgggca gctcacttaa gttcatgtcg ctgtccagct gctgagccac 34260

aggctgctgt ccaacttgcg gttgcttaac gggcggcgaa ggagaagtcc acgcctacat 34320

gggggtagag tcataatcgt gcatcaggat agggcggtgg tgctgcagca gcgcgcgaat 34380

aaactgctgc cgccgccgct ccgtcctgca ggaatacaac atggcagtgg tctcctcagc 34440

gatgattcgc accgcccgca gcataaggcg ccttgtcctc cgggcacagc agcgcaccct 34500

gatctcactt aaatcagcac agtaactgca gcacagcacc acaatattgt tcaaaatccc 34560

acagtgcaag gcgctgtatc caaagctcat ggcggggacc acagaaccca cgtggccatc 34620

ataccacaag cgcaggtaga ttaagtggcg acccctcata aacacgctgg acataaacat 34680

tacctctttt ggcatgttgt aattcaccac ctcccggtac catataaacc tctgattaaa 34740

catggcgcca tccaccacca tcctaaacca gctggccaaa acctgcccgc cggctataca 34800

ctgcagggaa ccgggactgg aacaatgaca gtggagagcc caggactcgt aaccatggat 34860

catcatgctc gtcatgatat caatgttggc acaacacagg cacacgtgca tacacttcct 34920

caagattaca agctcctccc gcgttagaac catatcccag ggaacaaccc attcctgaat 34980

cagcgtaaat cccacactgc agggaagacc tcgcacgtaa ctcacgttgt gcattgtcaa 35040

agtgttacat tcgggcagca gcggatgatc ctccagtatg gtagcgcggg tttctgtctc 35100

aaaaggaggt agacgatccc tactgtacgg agtgcgccga gacaaccgag atcgtgttgg 35160

tcgtagtgtc atgccaaatg gaacgccgga cgtagtcata tttcctgaag caaaaccagg 35220

tgcgggcgtg acaaacagat ctgcgtctcc ggtcctgccg cttagcgtgt tccgtgtgat 35280

agttcaagta caaccacact cttaagttgg tcaaaagaat gctggcttca gttgtaatca 35340

aaactccatc gcatctaatc gttctgagga aatcatccac ggtagcatat gcaaatccca 35400

accaagcaat gcaactggat tgtgtttcaa gcaggagagg agagggaaga gacggaagaa 35460

ccatgttaat ttttttattc caaaagatta tccaaaacct caaaatgaag atctattaag 35520

tgaacgcgct cccctccggt ggcgtggtca aactctacag ccaaagaaca gataatggca 35580

tttgtaagat gttgcacaat ggcttccaaa aggcaaacgg ccctcacgtc caagtggacg 35640

taaaggctaa acccttcagg gtgaatctcc tctataaaca ttccagcacc ttcaaccatg 35700

cccaaataat tctcatctcg ccaccttctc aatatatctc taagcaaatc ccgaatatta 35760

agtccggcca ttgtaaaaat ctgctccaga gcgccctcca ccttacagcc tcaagcagcg 35820

aatcatgatt gcaaaaattc aggttcctca cagacctgta taagattcaa aagcggaaca 35880

ttaacaaaaa taccgcgatc ccgtaggtcc cttcgcaggg ccagctgaac ataatcgtgc 35940

aggtctgcac ggaccagcgc ggccacttcc ccgccaggaa ccatgacaaa agaacccaca 36000

ctgattatga cacgcatact cggagctatg ctaaccagcg tagccccgat gtaagcttgt 36060

tgcatgggcg gcgatataaa atgcaaggtg ctgctcaaaa aatcaggcaa agcctcgcgc 36120

aaaaaagaaa gcacatcgta gtcatgctca tgcagataaa ggcaggtaag ctccggaacc 36180

accacagaaa aagacaccat ttttctctca aacatgtctg cgggtttctg cataaacaca 36240

aaataaaata acaaaaaaac atttaaacat tagaagcctg tcttacaaca ggaaaaacaa 36300

cccttataag cataagacgg actacggcca tgccggcgtg accgtaaaaa aactggtcac 36360

cgtgattaaa aagcaccacc gacagctcct cggtcatgtc cggagtcata atgtaagact 36420

cggtaaacac atcaggttga ttcacatcgg tcagtgctaa aaagcgaccg aaatagcccg 36480

ggggaataca tacccgcagg cgtagagaca acattacagc ccccatagga ggtataacaa 36540

aattaatagg agagaaaaac acataaacac ctgaaaaacc ctcctgccta ggcaaaatag 36600

caccctcccg ctccagaaca acatacagcg cttccacagc ggcagccata acagtcagcc 36660

ttaccagtaa aaaagaaaac ctattaaaaa aacaccactc gacacggcac cagctcaatc 36720

agtcacagtg taaaaaaggg ccaagtgcag agcgagtata tataggacta aaaaatgacg 36780

taacggttaa agtccacaaa aaacacccag aaaaccgcac gcgaacctac gcccagaaac 36840

gaaagccaaa aaacccacaa cttcctcaaa tcgtcacttc cgttttccca cgttacgtca 36900

cttcccattt taagaaaact acaattccca acacatacaa gttactccgc cctaaaacct 36960

acgtcacccg ccccgttccc acgccccgcg ccacgtcaca aactccaccc cctcattatc 37020

atattggctt caatccaaaa taaggtatat tattgatgat gttacatcgt taattaa 37077